(19) Europäisches Patentamt / European Patent Office / Office européen des brevets

(11) **EP 3 780 003 A1**

(12) **EUROPEAN PATENT APPLICATION**
published in accordance with Art. 153(4) EPC

(43) Date of publication:
**17.02.2021 Bulletin 2021/07**

(21) Application number: **18911739.3**

(22) Date of filing: **12.12.2018**

(51) Int Cl.:
**G16H 50/50** (2018.01)

(86) International application number:
**PCT/JP2018/045610**

(87) International publication number:
**WO 2019/187372 (03.10.2019 Gazette 2019/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2018 JP 2018068278**

(71) Applicant: **NEC Solution Innovators, Ltd.**
**Koto-ku, Tokyo 136-8627 (JP)**

(72) Inventors:
• **TANIGUCHI Atsushi**
**Tokyo 136-8627 (JP)**
• **TAMANO Hiroshi**
**Tokyo 136-8627 (JP)**

(74) Representative: **Betten & Resch**
**Patent- und Rechtsanwälte PartGmbB**
**Maximiliansplatz 14**
**80333 München (DE)**

(54) **PREDICTION SYSTEM, MODEL GENERATION SYSTEM, METHOD, AND PROGRAM**

(57)     The model generation device 60 of the present invention includes regularization parameter candidate setting means 61 that outputs a search set of regularization parameters in which a plurality of solution candidates are set, the solution candidates having at least mutually different values of a regularization parameter that affects a term of a strong regularization variable that is one or more variables specifically defined among the explanatory variables, model learning means that learns a prediction model corresponding to each of the plurality of solution candidates, accuracy evaluation means 62 that evaluates a prediction accuracy of each of the learned prediction models, transition evaluation means 63 that evaluates the number of defective samples, which is the number of samples for which a graph shape or the transition is not valid for each of the learned prediction models, and model determination means 64 that determines a prediction model used for progress prediction, based on the evaluation result regarding the prediction accuracy, and the evaluation result regarding the graph shape or the number of defective samples.

FIG. 20

**Description**

Technical Field

The present invention relates to a model generation system, a model generation method, and a model generation program for generating a prediction model. The present invention also relates to a prediction system that predicts a future state based on past data.

Background Art

For example, let us consider predicting a secular change of an inspection value of an employee or the like measured in a health checkup or the like, a disease onset probability of a lifestyle-related disease based on it, or the like, and giving advice to each employee regarding health. Specifically, let us consider a case where future state (secular change of an inspection value, a disease onset probability, etc.) when the current lifestyle habits continue for three years is predicted based on past health checkup results and data showing lifestyle habits at that time, and then, an industrial physician, an insurer, etc. propose (health-instruct) review of the lifestyle habits, etc., or the employee himself/herself self-checks it.

In that case, the following method can be considered as a method for obtaining the transition of the predicted value. First, learn a prediction model that obtains a predicted value one year ahead from past data. For example, learn a prediction model that in association with past actual values (inspection values) of a prediction target, uses training data indicating further past inspection values that can be correlated with the past actual values, the attributes (age, etc.) of the prediction target person, and lifestyle habits at that time, and then, uses a prediction target item after 1 year as a target variable and other items that can be correlated with it as explanatory variables. Then, with respect to the obtained prediction model, the process of inputting the explanatory variables and obtaining a predicted value one year ahead while changing a time point (prediction time point) at which the value to be predicted is obtained is repeated for several years. At this time, by keeping the items related to lifestyle habits among the explanatory variables constant, it is possible to obtain the transition of the predicted value when the current lifestyle habits are continued for three years.

Related to the prediction of diagnosis of lifestyle-related diseases, for example, there are prediction systems described in PTLs 1 and 2. The prediction system described in PTL 1 predicts the disease onset probability of a lifestyle-related disease using a plurality of neural networks that have learned the presence or absence of onset according to the same learning pattern consisting of six items of age, body mass index (BMI), diastolic blood pressure (DBP), HDL cholesterol, LDL cholesterol, and insulin resistance index (HOMA-IR).

Further, it is described that the prediction system disclosed in PTL 2, when predicting the medical expenses, the medical practice, and the inspection values of the next year from the medical expenses, the medical practice, the inspection values, and lifestyle habits of this year, creates a model by limiting the direction of the correlation between each data (the direction of edges in the graph structure). Specifically, as shown in Fig. 38B and Fig. 38C of PTL 2, lifestyle habits affect the inspection values, the inspection values affect the medical practice, the medical practice affects the medical expenses, and these states in the past will affect these states in the future. Further, PTL 2 describes that a model is created for each age.

Citation List

Patent Literature

PTL 1: Japanese Patent Application Laid-Open No. 2012-64087
PTL 2: Japanese Patent Application Laid-Open No. 2014-225175

Summary of Invention

Technical Problem

The problem is that when progress prediction is performed using a prediction model that has been learned by using all the explanatory variables that can be correlated to the prediction target in order to improve the prediction accuracy without any particular restriction, there are cases in which the transition of the predicted value in the progress prediction exhibits changes that are different from the common findings. For example, let us consider giving advice based on the transition of the predicted value obtained by predicting the disease onset probability of a lifestyle-related disease and the inspection values related to it when the explanatory variables related to lifestyle habits are constant.

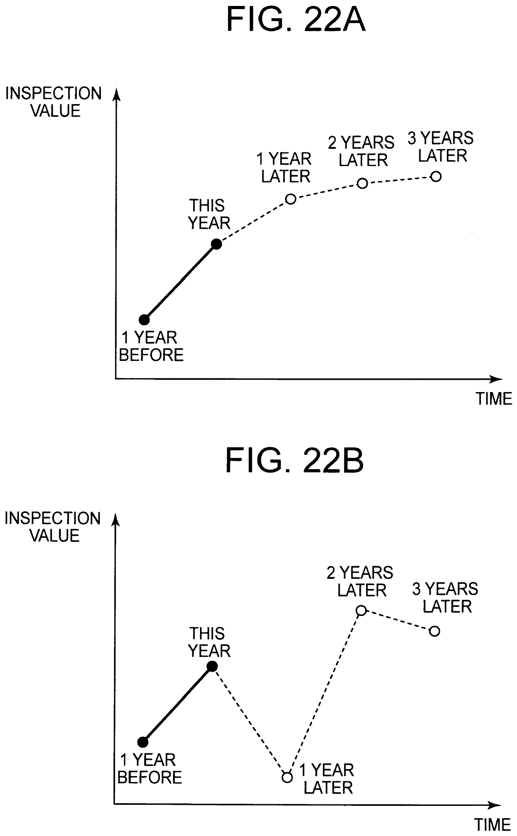

According to the general feeling, if the lifestyle habits are kept constant, for example, as shown in Fig. 22A, it is natural that the disease onset probability of a lifestyle-related disease and the transition of inspection values related thereto gradually approach some value.

However, if the progress prediction is performed simply by repeatedly applying the prediction model that predicts the predicted value at the next time point (for example, one year later) in a predetermined prediction time unit, although the lifestyle habits are kept constant, as shown in Fig. 22B, there is a possibility that the prediction result will be shown in the form of a bumpy graph that rises and falls with the direction of change (plus or minus) not fixed, such that it changes upwards in one year and downwards in another year. There is a problem that even if an advice is given to improve lifestyle habits based on such a prediction result, since both the person giving the advice and the person receiving the advice feel uncomfortable, the prediction result cannot be used as a basis for the advice.

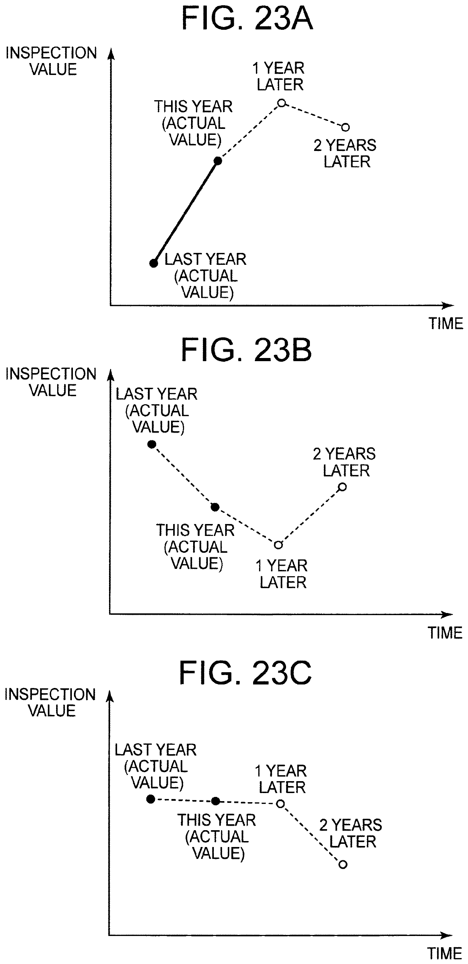

In addition, Fig. 23 is a graph showing another example of transition of the predicted value. Depending on the prediction target item such as inspection value prediction, for example, in the case of a graph shape in which the tendency of change changes with time as shown in Figs. 23A and 23B, or also in the case of a graph shape in which the magnitude of the change (the rising angle and the falling angle) greatly changes as shown in Fig. 23C, it can be cited as an example of a sample that cannot be interpreted. Here, the sample refers to an arbitrary combination of the values of the explanatory variables shown as prediction target data.

For example, like the above health simulation, when it is considered that an industrial physician, an insurer, etc. propose (health-instruct) review of the lifestyle habits based on the results of predicting the progress based on past health checkup results and data showing lifestyle habits at that time, or the employee himself/herself self-checks it, it is important for a prediction mechanism to reduce the number of samples in which the above-mentioned invalid transition of the predicted value is output. However, the prediction systems described in PTLs 1 and 2 do not consider the validity of the transition of the predicted value in the progress prediction.

For example, PTL 1 describes that by utilizing the variability of prediction results obtained from a plurality of neural networks having different constituent elements, it is possible to accurately obtain the disease onset probability of a lifestyle-related disease in a certain year in the future (specifically after six years). However, when the predicted value is obtained by such a prediction method, the transition of the predicted value does not always approach a certain value.

In addition, for example, PTL 2 describes that when predicting the medical expenses, the medical practice, and the inspection values of the next year from the medical expenses, the medical practice, the inspection values, and lifestyle habits of this year, it is possible to obtain a model that is intuitively easy to understand by limiting the direction of correlation between each data (the direction of edges in the graph structure). Further, PTL 2 discloses that a model is created for each age. However, when the transition of the predicted value is obtained by the prediction method described in PTL 2, there is a possibility that the transition of the predicted value may not approach a certain value.

Furthermore, the above problem is not limited to the case of predicting an inspection value that lifestyle habits influence and the disease onset probability of a lifestyle-related disease based on the inspection value, but similarly occurs in the case of predicting items having similar properties. In other words, for a certain item, when the transition of a value of the item when values of some items except the actual values (for example, items whose values can be controlled by a person) among the other items related to the item are set to constant values is considered, due to the characteristics of the item, the same problem occurs if the item has a valid transition type (pattern) such that the value of the item gradually approaches (converges) to a certain value, diverges, the direction of change is constant, gradually diverges while changing the direction of change, gradually converges while changing the direction of change. In addition, "valid" here means probable at least in the knowledge of the person who handles the predicted value.

Therefore, it is an object of the present invention to provide a model generation system, a prediction system, a model generation method, and a model generation program that can reduce the number of samples in which an invalid transition of a predicted value is output in progress prediction.

Solution to Problem

A model generation system according to the present invention includes: regularization parameter candidate setting means that outputs a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model; model learning means that learns, using training data, a prediction model corresponding to each of the plurality of solution candidates included in the search set; accuracy evaluation means that evaluates, using predetermined verification data, a prediction accuracy of each of a plurality of the learned prediction models; transition evaluation means that evaluates, for each of the plurality of the learned prediction models, a graph shape indicated by a transition of a predicted value obtained from the prediction model or a number of defective samples, which is a number of samples for which the transition is not valid, using predetermined verification data; and model determination means that determines a prediction model used for the progress prediction from among the plurality of the learned prediction models based on an evaluation result regarding the prediction accuracy and an evaluation result regarding the graph shape or the number of defective samples.

Further, the model generation system according to the present invention may include: constrained model evaluation means that evaluates, using predetermined verification data, a prediction accuracy of a constrained model, which is one of prediction models used for progress prediction performed by fixing some values of a plurality of explanatory variables, which is a prediction model that predicts a value of a prediction target item at a prediction time point when a predicted value is obtained, and which is a prediction model in which at least a constraint that a variable other than a main variable indicating a value of the prediction target item at a prediction reference point is not used as a non-control variable that is an explanatory variable whose value changes in the progress prediction is imposed to the explanatory variable; regularization parameter candidate setting means that outputs a search set which is a search set of regularization parameters used for regularization of a calibration model, and in which a plurality of solution candidates are set, the solution candidates having at least mutually different values of a regularization parameter that affects a term of a strong regularization variable that is one or more variables specifically defined among the explanatory variables used for a model formula of the calibration model, the calibration model being one of the prediction models used for the progress prediction, the calibration model being a prediction model for predicting a calibration value for calibrating the predicted value obtained in the constrained model for arbitrary prediction target data, and the calibration model being a prediction model including two or more non-control variables and one or more control variables that can be controlled by a person in the explanatory variables; model learning means that learns, using training data, a calibration model corresponding to each of the plurality of solution candidates included in the search set; accuracy evaluation means that evaluates, using predetermined verification data, a prediction accuracy of each of the plurality of learned calibration models; transition evaluation means that evaluates, for each of the plurality of learned calibration models, a graph shape indicated by a transition of the predicted value after calibration obtained as a result of calibrating the predicted value obtained from the constrained model with the calibration value obtained from the calibration model or the number of defective samples, which is the number of samples for which the transition is not valid, using predetermined verification data; and model determination means that determines a calibration model used for the progress prediction from among the plurality of learned calibration models based on an index regarding the prediction accuracy and an index regarding the graph shape or the number of defective samples.

Further, a prediction system according to the present invention includes: regularization parameter candidate setting means that outputs a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model; model learning means that learns, using training data, a prediction model corresponding to each of the plurality of solution candidates included in the search set; accuracy evaluation means that evaluates, using predetermined verification data, a prediction accuracy of each of a plurality of the learned prediction models; transition evaluation means that evaluates, for each of the plurality of the learned prediction models, a graph shape indicated by a transition of a predicted value obtained from the prediction model or a number of defective samples, which is a number of samples for which the transition is not valid, using predetermined verification data; model determination means that determines a prediction model used for the progress prediction from among the plurality of the learned prediction models based on an evaluation result regarding the prediction accuracy and an evaluation result regarding the graph shape or the number of defective samples; model storage means that stores a prediction model used for the progress prediction; and prediction means that when prediction target data is given, performs the progress prediction using the prediction model stored in the model storage means.

A model generation method according to the present invention includes: outputting a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model; learning, using training data, a prediction model corresponding to each of the plurality of solution candidates included in the search set; evaluating, for each of the plurality of the learned prediction models, each of a prediction accuracy and a graph shape indicated by a transition of a predicted value obtained from the prediction model or a number of defective samples, which is a number of samples for which the transition is not valid, using predetermined verification data; and determining a prediction model used for the progress prediction from among the plurality of the learned prediction models based on an evaluation result regarding the prediction accuracy and an evaluation result regarding the graph shape or the number of defective samples.

A model generation program according to the present invention causes a computer to execute the processes of: outputting a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model; learning, using training data, a prediction model corresponding to each of the plurality of solution candidates included in the search set; evaluating, for each of the plurality of the learned prediction models, each of a prediction accuracy and a graph shape indicated by a transition of a predicted value obtained from the prediction model or a number of defective samples, which is a number of samples for which the transition is not valid, using predetermined verification data; and determining a prediction model used for the progress prediction from among the plurality of the learned prediction models based on an evaluation result regarding the prediction accuracy and an evaluation result regarding the graph shape or the number of defective samples.

Advantageous Effects of Invention

According to the present invention, it is possible to reduce the number of samples in which a transition of a predicted value that is not valid in progress prediction is output.

Brief Description of Drawings

[Fig. 1] It depicts an explanatory diagram showing an example of a reference point, an evaluation target period, a prediction time point, and a prediction unit time in progress prediction.
[Fig. 2] It depicts a conceptual diagram of a prediction model.
[Fig. 3] It depicts is a block diagram showing a configuration example of a prediction system of a first exemplary embodiment.
[Fig. 4] It depicts a flowchart showing an operation example of a model learning phase of the prediction system of the first exemplary embodiment.
[Fig. 5] It depicts a flowchart showing an operation example of a prediction phase of the prediction system of the first exemplary embodiment.
[Fig. 6] It depicts an explanatory diagram showing an example of curve fitting in progress prediction.
[Fig. 7] It depicts an explanatory diagram showing an example of transition vectors and transition angles in progress prediction.
[Fig. 8] It depicts a conceptual diagram showing an effect of setting a regularization parameter of the first exemplary embodiment.
[Fig. 9] It depicts an explanatory diagram showing an example of a simplified expression of a prediction formula of a prediction model.
[Fig. 10] It depicts a block diagram showing another configuration example of the prediction system of the first exemplary embodiment.
[Fig. 11] It depicts a block diagram showing a configuration example of a prediction system of a second exemplary embodiment.
[Fig. 12] It depicts an explanatory diagram showing an example of a graph shape.
[Fig. 13] It depicts a block diagram showing a configuration example of a model application unit of the second exemplary embodiment.
[Fig. 14] It depicts a flowchart showing an operation example of a model learning phase of the prediction system of the second exemplary embodiment.
[Fig. 15] It depicts a flowchart showing an operation example of a prediction phase of the prediction system of the second exemplary embodiment.
[Fig. 16] It depicts a block diagram showing another configuration example of the prediction system of the second exemplary embodiment.
[Fig. 17] It depicts a block diagram showing a configuration example of a health simulation system of a third exemplary embodiment.
[Fig. 18] It depicts an explanatory diagram showing an example of a method of changing an inquiry item and a method of displaying a prediction result by a simulation unit.
[Fig. 19] It depicts a schematic block diagram showing a configuration example of a computer according to each exemplary embodiment of the present invention.
[Fig. 20] It depicts a block diagram showing an outline of a model generation device of the present invention.

[Fig. 21] It depicts a block diagram showing an outline of the prediction system of the present invention.
[Fig. 22] It depicts a graph showing an example of transition of predicted values.
[Fig. 23] It depicts a graph showing an example of transition of predicted values.

Description of Embodiments

An exemplary embodiment of the present invention will be described below with reference to drawings. First, terms used in the present invention will be described. Hereinafter, the time point when the progress prediction is started and at least having the actual value is referred to as a "reference point". Note that the reference point may be the latest time point having the actual value. Further, hereinafter, the period from the reference point to the earliest prediction time point when the change over time is desired may be referred to as an "evaluation target period for progress prediction" or simply "evaluation target period".

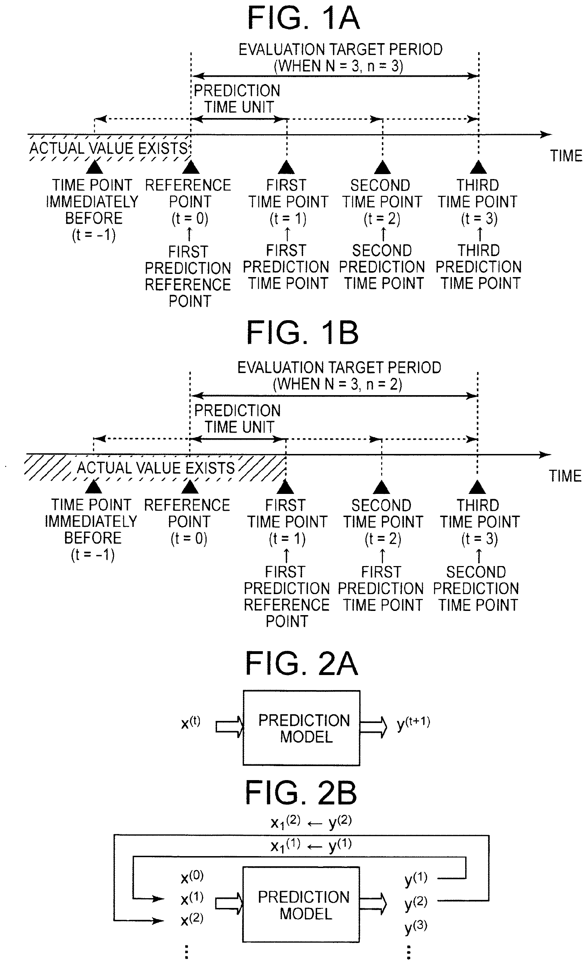

Fig. 1 is an explanatory diagram showing an example of a reference point, an evaluation target period, a prediction time point, and a prediction time unit in the progress prediction. As shown in Figs. 1A and 1B, in the progress prediction, the time point one ahead in the predetermined prediction time unit from the reference point is called a first time point, and the time point two ahead is called a second time point,... At this time, if the time point is a future time point, that is, a time point when the predicted value is desired to obtain, the time point is called a prediction time point. In the example shown in Fig. 1A, the first time point, the second time point, and the third time point are called a first prediction time point, a second prediction time point, and a third prediction time point, respectively. It should be noted that the prediction time unit may be regarded as a standard time interval in which the prediction model can output a predicted value in one prediction. The prediction time unit is a unit of iteration of the prediction model in the progress prediction. In addition, from the viewpoint of prediction accuracy, it is preferable that the prediction time unit in the progress prediction is constant, but it is not necessarily constant. Also, from an arbitrary time point (the n-th prediction time point) when a predicted value is desired to obtain, by tracing back the prediction time unit, it is also possible to define the n-1 th prediction time point, the n-2 th prediction time point,...., and the reference point.

For example, when the predicted value is obtained every year, the prediction time unit may be approximately one year (one year $\pm \alpha$). As the prediction time point itself, an arbitrary time point can be designated regardless of the prediction time unit that is a repeating unit of the prediction model. Specifically, if the value at a certain time point $t_p$ is desired to predict, it may be predicted by using an actual value at the time point $t_p$-$\Delta t$ (the time point that the prediction time unit goes back from the prediction time point) or a predicted value corresponding thereto, and a value indicating the prediction condition at the time point $t_p$ (for example, items related to lifestyle habits at the prediction time point). Here, $\Delta t$ corresponds to the prediction time unit.

Hereinafter, a time point (the above-mentioned $t_p$-$\Delta t$) traced back by the prediction time unit with respect to an arbitrary prediction time point (the above-mentioned $t_p$) may be referred to as a prediction reference point. Further, the prediction reference point corresponding to the first prediction time point (that is, the prediction time point closest to the reference point) included in the evaluation target period may be referred to as a first prediction reference point. It should be noted that the first prediction reference point is a starting time point in the iteration of the prediction model. Therefore, it can be said that the progress prediction predicts the value of the prediction target item at each prediction time point included in the evaluation target period based on the information at the first prediction reference point. Furthermore, the prediction model used for the progress prediction can be said to be a model that predicts the value of the prediction target item at the prediction time point that is one ahead of the prediction reference point, based on the information at the prediction reference point. In the following, the number of time points (excluding the reference point) included in the evaluation target period may be expressed as N, and the number of prediction time points may be expressed as n.

Note that, as shown in Fig. 1B, the reference point and the first reference point do not necessarily have to match. Further, for example, when the reference point is t = 0, for example, even if only one prediction time point is included in the evaluation target period (N ≥ 2) of t to t + N, obtaining the transition of the value of the prediction target item in the evaluation target period is referred to as "progress prediction". In this case, the actual prediction is only once (that is, n = 1). In other words, the "progress prediction" may obtain a predicted value at each prediction time point included in a period including two or more time points (referring to a time point in the prediction time unit) from the reference point and a period (evaluation target period) including at least one prediction time point (that is, a time point when the predicted value is obtained). In addition, the "progress prediction" may be expressed as obtaining the transition of the value of the prediction target item in the period by obtaining the predicted value at each prediction time point in this way. Of course, the evaluation target period may include two or more prediction time points.

Next, the explanatory variable in the present invention will be described. The following formula (1) is an example of a prediction model formula (prediction formula) when a linear model is used as the prediction model. Although a linear model is shown as an example of the prediction model for simplification of description, the prediction model is not particularly limited to a linear model, and for example, a piecewise linear model used for heterogeneous mixture learning, a neural network model, or the like may be used.

$$y = a_0 + a_1 x_1 + a_2 x_2 + \ldots + a_m x_m \quad \ldots \quad (1)$$

In formula (1), y is a target variable and $x_i$ is an explanatory variable (where i = 1,..., m). Note that m is the number of explanatory variables. Further, $a_i$ (where i = 0,..., m) is a parameter of the linear model, $a_0$ is an intercept (constant term), and $a_i$ is a coefficient of each explanatory variable.

First, consider a case where the prediction target is one inspection item. At this time, the prediction model used for the progress prediction can be considered as a function that outputs a value $y^{(t)}$ of the prediction target item at a time point t using, as an input, a combination of explanatory variables $x^{(t-1)} = \{x_1^{(t-1)}, x_2^{(t-1)},..., x_m^{(t-1)}\}$ that can be acquired at a prediction reference point (t - 1) that is one time point before the time point t as the prediction time point. The number in parentheses on the right shoulder of y, x represents time on the prediction unit time axis. Fig. 2A is a conceptual diagram of such a prediction model. In Fig. 2A, the prediction reference point is shown as the time point t and the prediction time point is shown as the time point (t + 1), but if t is regarded as an arbitrary numerical value, it is substantially the same as the above.

Furthermore, in the progress prediction, in order to predict the value $y^{(t + 1)}$ of the prediction target item at the time point (t + 1), as one of the explanatory variables, a variable indicating the value $y^{(t)}$ of the prediction target item at the prediction reference point (t), or a variable calculated from the value $y^{(t)}$ is used. Here, if the explanatory variable is $x_1$, the relationship between the two can be expressed as $x_1^{(t)} \leftarrow y^{(t)}$. Note that $x_1^{(t)} \leftarrow y^{(t)}$ shows that the value $y^{(t)}$ of the prediction target item at a certain time point t is used for one of the explanatory variables (specifically, $x_1^{(t)}$ which is the value of the prediction target item at the time point t) in the prediction model for predicting the value $y^{(t + 1)}$ of the prediction target item at the next time point (t + 1). Then, the prediction model in the progress prediction can be more simply considered as a function that outputs, using, as an input, the combination $X^{(t)}$ of the explanatory variables at a time point t, a predicted value ($y^{(t + 1)}$) corresponding to one of the explanatory variables, for example $x_1^{(t + 1)}$, at the next time point (t + 1) as the predicted value.

Using such a property, in the progress prediction, a combination $X^{(0)}$ of explanatory variables including the explanatory variable $x_1^{(0)}$ corresponding to the value $y^{(0)}$ of the prediction target item at the prediction reference point (t = 0) is input to the prediction model to obtain the value $y^{(1)}$ of the prediction target item at the first prediction time point (t = 1). Then, the combination $X^{(1)}$ of the explanatory variables at the first prediction time point including $x_1^{(1)}$ is generated from the $y^{(1)}$ and input to the prediction model, and a value $y^{(2)}$ of the prediction target item at the second prediction time point (t = 2) is obtained. After that, similar processing is repeated until the value at the final prediction time point is obtained (see Fig. 2B). In other words, in the progress prediction, a process of obtaining the value of the prediction target item at the prediction time point by inputting the combination of the explanatory variables at the prediction reference point into the prediction model, while updating the prediction reference point and the prediction time point may be repeated n times (that is, it may be repeated until a predicted value after n prediction time units is obtained). Below, the procedure of the progress prediction is expressed using the prediction formula of the formula (1).

Predicted value at the first prediction time point: $y^{(1)} = a_0 + a_1 x_1^{(0)} + \ldots + a_m x_m^{(0)}$

Predicted value at the second prediction time point: $y^{(2)} = a_0 + a_1 x_1^{(1)} + \ldots + a_m x_m^{(1)}$

$$\vdots$$

Predicted value at the n-th prediction time point: $y^{(n)} = a_0 + a_1 x_1^{(n)} + \ldots + a_m x_m^{(n)}$

However, $x_1^{(t)} \leftarrow y^{(t)}$.

Note that, in the above description, the example in which the progress prediction is performed for one inspection item using the prediction model has been shown, but the number of prediction target items is not limited to one. That is, it is considered that the predicted values of a plurality of inspection items and their transitions are obtained using a prediction formula learned for each inspection item. These prediction formulas can be simply expressed as follows. Although a linear model is illustrated as the prediction model in this example as well, the prediction model is not limited to the linear model.

$$y_1^{(t+1)} = a_{10} + a_{11}x_1^{(t)} + a_{12}x_2^{(t)} + \ldots + a_{1m}x_m^{(t)}$$

$$y_2^{(t+1)} = a_{20} + a_{21}x_1^{(t)} + a_{22}x_2^{(t)} + \ldots + a_{2m}x_m^{(t)}$$

$$\vdots$$

$$y_u^{(t+1)} = a_{u0} + a_{u1}x_1^{(t)} + a_{u2}x_2^{(t)} + \ldots + a_{um}x_m^{(t)}$$

$$\ldots \quad (2)$$

Note that u in formula (2) is the number of inspection items, and u < m. In each prediction formula, $x_k^{(t)}$ (where k = 1 to u) is an explanatory variable corresponding to the inspection item 1 to u. This shows that not only the past value of the inspection item to be predicted by itself, but also the past values of other inspection items can be used as explanatory variables, and whether they are actually used or not is adjusted by the coefficients of the variables.

In the following, $x_i^{(t+1)} = y_i^{(t+1)}$ (where i = 1 to u, u < m). Then, the above formula (2) can be written as the following formula (3). In addition, a formula (4) is the matrix notation of formula (3).

$$x_1^{(t+1)} = a_{10} + a_{11}x_1^{(t)} + a_{12}x_2^{(t)} + \ldots + a_{1m}x_m^{(t)}$$

$$x_2^{(t+1)} = a_{20} + a_{21}x_1^{(t)} + a_{22}x_2^{(t)} + \ldots + a_{2m}x_m^{(t)}$$

$$\vdots$$

$$x_u^{(t+1)} = a_{u0} + a_{u1}x_1^{(t)} + a_{u2}x_2^{(t)} + \ldots + a_{um}x_m^{(t)}$$

$$\ldots \quad (3)$$

[Math 1]

$$\begin{pmatrix} x_1 \\ \vdots \\ x_u \end{pmatrix}^{(t+1)} = \begin{pmatrix} a_{10} & \cdots & a_{1m} \\ \vdots & \ddots & \vdots \\ a_{u0} & \cdots & a_{um} \end{pmatrix} \begin{pmatrix} 1 \\ x_1 \\ \vdots \\ x_m \end{pmatrix}^{(t)} \quad \cdots \quad (4)$$

In the formula (4), the prediction formulas for a plurality of inspection items are collectively shown as one formula using matrix, but actually, as shown in the formula (2), the prediction formula is retained for each inspection item and without omission of explanatory variables.

It is also possible to simplify the above formula (4) and write it as the formula (5). Note that $X^{(t)}$ and $X^{(t+1)}$ are column vectors, and explanatory variables $x_k^{(t)}$ (k = u + 1 to m) corresponding to items (inquiry items, etc.) other than inspection items among explanatory variables shall contain the specified values.

$$X^{(t+1)} = AX^{(t)} \quad \ldots \quad (5)$$

At this time, the predicted value at the n-th prediction time point (t = n) obtained based on the data at the reference point (t = 0) is expressed as follows. In addition, $A^n$ represents the n-th power of A.

Predicted value at the first prediction time point: $X^{(1)} = AX^{(0)}$

Predicted value at the second prediction time point: $X^{(2)} = A^2X^{(1)} = AAX^{(0)}$

$$\vdots$$

Predicted value at the nth prediction time point: $X^{(n)} = A^nX^{(0)} \quad \ldots \quad (6)$

In the above description, the prediction formula for predicting the predicted value $X^{(n)}$ at the time point +n is defined as formula (6) using the data $X^{(0)}$ at the reference point. Also in the progress prediction, the predicted value at a desired time point may be acquired at a pinpoint, for example, by using a prediction formula such as the formula (6) for directly obtaining the predicted value at the +n time point.

In the above description, $x_1^{(t)}$ and $y_i^{(t)}$ do not have to be exactly the same value (the same applies to $x_1^{(t)}$ and $y^{(t)}$ when there is one inspection item). For example, $x_1^{(t)} = y_i^{(t)} + \alpha$, $x_1^{(t)} = \beta y_i^{(t)}$, $x_i^{(t)} = (y_i^{(t)})^\gamma$, $x_1^{(t)} = \sin(y_i^{(t)})$ and the like (where $\alpha$, $\beta$, and $\gamma$ are all coefficients). That is, $x_1^{(t)}$ as an explanatory variable may be a variable indicating the value $y_i^{(t)}$ of the prediction target item at that time point (prediction reference point). Hereinafter, the variable corresponding to the above $x_1^{(t)}$ may be referred to as "main variable". More specifically, the main variable is a variable indicating the value of the prediction target item at the prediction reference point.

The explanatory variables may include variables other than the main variable. Hereinafter, an explanatory variable whose value is made not to change in the progress prediction, that is, an explanatory variable that can be controlled by a person, like the variable indicating the item related to the above-mentioned lifestyle habits, is referred to as a "control variable". In the following, among explanatory variables, explanatory variables other than "control variables", that is, explanatory variables whose values change in progress prediction may be referred to as "non-control variables". Examples of non-control variables include the above main variable, a variable that indicates the value of the prediction target item at an arbitrary time point before the prediction reference point, a variable that indicates an arbitrary statistic (difference, etc.) calculated from that variable and other variables, a variable represented by one-variable function or other-variable function based on those variables, and arbitrary other variables (variables indicating values of items other than the prediction target item at the prediction time point or an arbitrary time point before that), etc.

If the explanatory variables of the prediction model include non-control variables (hereinafter referred to as sub-variables) other than the main variable, when the prediction is repeated, in addition to the above $x_1^{(t)} \leftarrow y_i^{(t)}$, the value of each sub-variable may also be $x_k^{(t)} \leftarrow (y_i^{(t)},...)$. Here, $x_k^{(t)}$ represents an arbitrary sub-variable, and $y_i^{(t)}$ represents a variable constituting the sub-variable and an arbitrary variable that can be acquired at the prediction reference point. As a result of model learning, there may be a case where the main variable is not used (coefficient is zero), but only the sub-variable and the control variable are used.

By the way, in general, learning of a prediction model is performed with emphasis on prediction accuracy. In such a prediction accuracy-emphasized learning method (hereinafter referred to as the first method), for example, an optimum solution of the model is obtained by searching for a solution of a model parameter that minimizes a predefined error function. However, since the method does not consider the graph shape in the progress prediction as described above, there is a possibility that many samples that cannot be interpreted when used in the progress prediction are output.

As one of the solutions to the above problem, if the explanatory variable used in the prediction model is limited, and if some kind of constraint is added to the coefficient of the explanatory variable, it is possible to forcibly fit the transition of the predicted value in the progress prediction to a predetermined graph shape (hereinafter referred to as a second method). However, in the second method that emphasizes fitting to a desired graph shape, it is necessary to limit the number of explanatory variables whose values are changed during the progress prediction to one, and there is a problem that the prediction accuracy decreases.

For example, when the latest value of the prediction target item (latest inspection value) is used as the explanatory variable, other inspection values (the inspection value at the two previous time points or the inspection value of other than the prediction target item) or the statistic values using the other inspection values cannot be used as the explanatory variables of the prediction model. Therefore, depending on the nature of the prediction target item and how to select other explanatory variables, it is not possible to properly express the relationship between the target variable (predicted value) and the explanatory variables in the prediction model, and there is a risk that the prediction accuracy may decrease.

In the following exemplary embodiments, a method for reducing the number of samples in which an invalid transition of a predicted value is output in progress prediction will be described without limiting the explanatory variables.

First exemplary embodiment

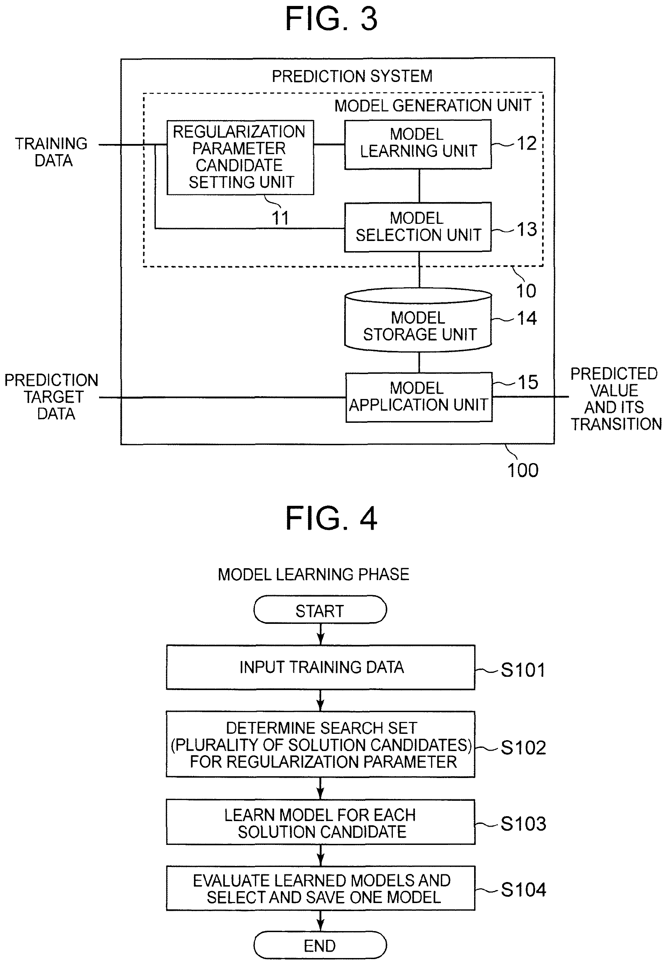

Fig. 3 is a block diagram showing a configuration example of a prediction system of the first exemplary embodiment. A prediction system 100 shown in Fig. 3 includes a model generation unit 10, a model storage unit 14, and a model application unit 15.

The model generation unit 10 is a processing unit that generates a prediction model used for progress prediction when training data is input and stores the prediction model in the model storage unit 14, and includes a regularization parameter candidate setting unit 11, a model learning unit 12, and a model selection unit 13.

The regularization parameter candidate setting unit 11, when the training data is input, outputs, as a search set of regularization parameters that are parameters used for regularization of the prediction model, a search set in which a plurality of solution candidates are set, the solution candidates having different values of a regularization parameter that affects a term of at least one or more variables (hereinafter, referred to as strong regularization variables) specifically defined among the explanatory variables.

The regularization is generally performed in order to prevent overlearning and increase generalization capability during model learning, and prevents the value of a model coefficient (coefficient of explanatory variable of the prediction formula) from becoming a large value by adding a penalty term to the error function. Here, the strong regularization variable is a variable that imparts stronger regularization than other explanatory variables. Further, in the following, among the explanatory variables, variables other than "strong regularization variable" may be referred to as "weak regularization variable".

In the present exemplary embodiment, the explanatory variables are classified as follows according to their uses and properties.

[Classification of explanatory variables]

(1) Control variable or non-control variable (including main variable and sub variable)
(2) Strong regularization variable or weak regularization variable

The strong regularization variable may be, for example, one or more variables specifically defined among variables (sub variables and control variables) other than the main variable. Further, the strong regularization variable may be, for example, one or more variables specifically defined among variables (that is, sub variables) other than the main variable among the two or more non-control variables included therein. In the following, an example in which among the explanatory variables, a variable indicating the value of the prediction target item at an arbitrary time point before the prediction reference point (one time point before in a predetermined prediction unit time from the prediction time point as the time point when the predicted value is obtained) or a variable indicating an arbitrary statistic calculated from that variable and other variables is used as a strong regularization variable is shown, but the strong regularization variable is not limited to these.

For example, when the explanatory variables used for the prediction model used in the progress prediction for a certain inspection item within the next three years include (a) the inspection value of last year (non-control variable (main variable)), (b) the difference value between the inspection value of the year before last and the inspection value of last year (non-control variable (sub-variable)), and (c) one or more variables (control variables) corresponding to one or more items related to lifestyle habits, the difference value of (b) may be used as the "strong regularization variable". The strong regularization variable is not limited to one.

The model learning unit 12 uses the training data to respectively learn the prediction models corresponding to the solution candidates included in the search set of regularization parameters output from the regularization parameter candidate setting unit 11. That is, the model learning unit 12 learns, for each solution candidate of the regularization parameter output from the regularization parameter candidate setting unit 11, a prediction model corresponding to the solution candidate by using the training data.

The model selection unit 13 selects a prediction model that has a high prediction accuracy and has a small number of samples (hereinafter referred to as the number of defective samples) that cannot be interpreted from among a plurality of prediction models (learned models having mutually different values of the regularization parameters that affect the term of the strong regularization variable) learned by the model learning unit 12. The model selection unit 13 may use, for example, predetermined verification data (for example, a data set including a combination of explanatory variables whose target values are known) to calculate the prediction accuracy and the number of defective samples of each learned prediction model and select a predetermined number of prediction models to be used for progress prediction based on the calculated prediction accuracy of each prediction model and the number of defective samples. The number of models to be selected may be one or more. The model selection method by the model selection unit 13 will be described later. The verification data may be the training data used for learning or a part thereof (data divided for verification).

The model storage unit 14 stores the prediction model selected by the model selection unit 13.

When the prediction target data is input, the model application unit 15 uses the prediction model stored in the model storage unit 14 to perform progress prediction. Specifically, the model application unit 15 may apply the prediction target data to the prediction model stored in the model storage unit 14 to calculate the predicted value (value of the prediction target item) at each prediction time point included in the evaluation target period.

Next, the operation of the prediction system of the present exemplary embodiment will be described. The operation of the prediction system of the present exemplary embodiment is roughly divided into a model learning phase in which a prediction model is learned using training data and a prediction phase in which progress prediction is performed using the learned prediction model.

First, the model learning phase will be described. The model learning phase is performed at least once before the prediction phase. Fig. 4 depicts a flowchart showing an operation example of the model learning phase of the prediction system of the present exemplary embodiment.

In the example shown in Fig. 4, first, training data is input to the prediction system (step S101). Here, the input training data may be a data set in which the value of a prediction target item (target value) which is the target variable in the prediction model, and a plurality of variables (more specifically, a combination of these plurality of variables) that can be correlated with the value of the prediction target item and are explanatory variables in the prediction model are associated with each other. In this example, the value of the prediction target item (target value of the prediction model) at a certain time point t', and a data set indicating a combination of variables including the non-control variables (main variable and sub variables) and the control variables when the time point (t'-1) immediately before that time point t' is used as the prediction reference point are input. The time point t' is not particularly limited as long as it is a time point when the target value is obtained, and it is possible to specify two or more different time points in the training data.

In step S101, the conditions of the regularization parameter and the like may be accepted together with the training data.

Next, the regularization parameter candidate setting unit 11 outputs a search set in which a plurality of solution candidates having different values of the regularization parameter that affects at least the term of the strong regularization variable among the explanatory variables of the prediction model are set (step S102).

Next, the model learning unit 12 uses the training data to respectively learn the prediction models corresponding to the solution candidates of the regularization parameter output from the regularization parameter candidate setting unit 11 (step S 103).

Next, the model selection unit 13 selects a prediction model having a high prediction accuracy and a small number of defective samples from among the plurality of learned prediction models, and stores it in the model storage unit 14 (step S104).

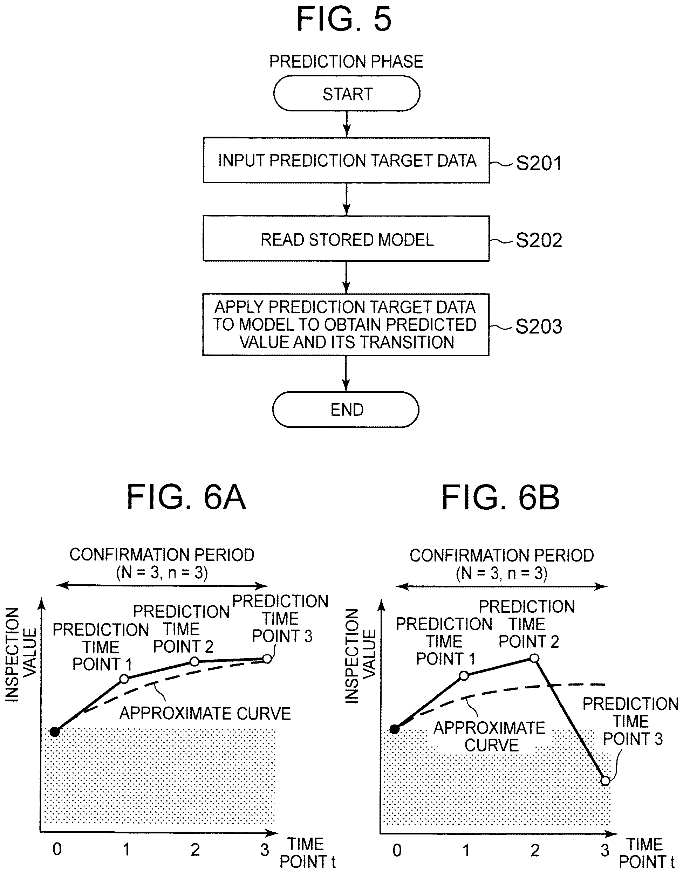

Next, the prediction phase will be described. Fig. 5 is a flowchart showing an operation example of the prediction phase of the prediction system of this exemplary embodiment. The prediction phase may be performed, for example, every time a progress prediction is requested.

In the example shown in Fig. 5, first, prediction target data is input to the prediction system (step S201). The prediction target data input here may be data indicating an arbitrary combination of a plurality of variables that are the explanatory variables in the prediction model stored in the model storage unit 14. In this example, data indicating a combination of variables including non-control variables (main variable and sub variables) when the time point immediately before the first prediction time point (first prediction time point) in the evaluation target period of the current progress prediction is a prediction reference point (first prediction reference point) and control variables are input. The value of the control variable is arbitrary. At this time, it is also possible to specify the value of a control variable used for prediction at each prediction time point as the prediction condition.

When the prediction target data is input, the model application unit 15 reads the prediction model stored in the model storage unit 14 (step S202), applies the prediction target data to the read prediction model, and obtains a predicted value at each prediction time point included in the evaluation target period (step S203).

Next, a method of determining a search set of regularization parameters by the regularization parameter candidate setting unit 11 (a method of setting a plurality of solution candidates) will be described. In the following, a regularization parameter used for a linear model will be described as an example, but the regularization parameter is not limited thereto. Now, assume that a formula (7) is given as the prediction formula of the prediction model.
[Math 2]

$$y(x, w) = w_0 + w_1 x_1 + w_2 x_2 + \cdots + w_M x_M \quad \cdots \quad (7)$$

For the above prediction formula, if the target value is $t_n$ (where n = 1 to N), the error function for the above prediction formula is expressed as in a following formula (8a) or formula (8b), for example. Here, a target value $t_k$ corresponds to correct solution data (actual value, etc.) in the combination of the explanatory variables $\{x_1,..., x_M\}$ given by K pieces of training data. In the formulas (8a) and (8b), the second term on the right side corresponds to the penalty term.
[Math 3]

$$j(w) = \sum_{k=1}^{K} (t_k - y_k)^2 + \sum_{j=1}^{M} \lambda_j |w_j|^q \quad \cdots \quad (8a)$$

$$j(w) = \sum_{k=1}^{K}(t_k - y_k)^2 + \lambda \sum_{j=1}^{M}|w_j|^q \quad \cdot\cdot\cdot \quad (8\ b)$$

Here, the regularization parameter corresponds to parameters $\lambda$ and $\lambda_j$ (where j = 1 to M) used in the penalty term of the error function. Note that "$\|^q$" in the formula represents a norm, and for example, q = 1 (L1 norm) is used in the Lasso method and q = 2 (L2 norm) is used in the Ridge regression method. The above description is an example of the regularization parameter used for the linear model, but the regularization parameter is not limited to this.

Usually, the regularization parameter is determined by searching for a solution of $\lambda = \{\lambda_1,... \lambda_M\}$ (hereinafter referred to as the minimum solution) that minimizes the output J(w) of the error function including the penalty term. At this time, $\lambda$ may be set to one value for all the coefficients, or may be set to a different value for each coefficient. Note that if zero is set for all the coefficients, it means that regularization is not performed. In either case, one solution is determined for $\lambda$.

The regularization parameter candidate setting unit 11 of the present exemplary embodiment performs the following regularization parameter candidate setting process in place of or in addition to the normal regularization process. That is, the regularization parameter candidate setting unit 11 sets a plurality of solution candidates $\lambda'$, $\lambda''$, $\lambda'''$,... having different values for the regularization parameter that affects at least the term of the strong regularization variable (in this example, the difference variable $x_{diff}$) among the terms included in the prediction formula of the prediction model, and outputs them as a search set of the regularization parameter $\lambda$. Note that, each solution candidate may be one regularization parameter $\lambda$ that is commonly set for all the terms included in the prediction formula, or may be a set of a regularization parameter $\lambda_j$ that is set for each of the terms included in the prediction formula.

For example, assume that the strong regularization variable is $x_2$ in the prediction formula (7). In that case, when the error function is expressed by, for example, the formula (8a), the regularization parameter candidate setting unit 11 sets a plurality of solution candidates $\lambda^{(1)}$, $\lambda^{(2)}$,... having different values at least for the regularization parameter $\lambda_2$ corresponding to the term of the $x_2$. The number on the right shoulder is an identifier of the solution candidate. At this time, the regularization parameter $\lambda_{j'}$ (other than j' = 2) corresponding to the other terms may be an arbitrary value such as the minimum solution value or zero. It should be noted that the arbitrary value includes a value specified by the user, a value of a solution other than the minimum solution obtained as a result of adjustment by a processing unit other than the regularization parameter candidate setting unit 11 of the present exemplary embodiment by another method, or the like. Further, for example, when the error function is expressed by the formula (8b), the regularization parameter candidate setting unit 11 sets a plurality of solution candidates $\lambda^{(1)}$, $\lambda^{(2)}$,... having different values at least for the regularization parameter $\lambda$ that affects the term of the $x_2$.

Incidentally, the regularization parameter candidate setting unit 11, when determining the search set of regularization parameters, in each solution candidate, not only sets a fixed value for other regularization parameters (e.g., regularization parameters corresponding to terms other than the term of the strong regularization variable), but also can set different values for other regularization parameters. The regularization parameter candidate setting unit 11 may set the regularization parameter that affects the term of the strong regularization variable and the other regularization parameters at the same time, or can set these separately. In any case, it is assumed that the solution candidates differ in at least the value of the regularization parameter that affects the term of the strong regularization variable.

When the strong regularization variable includes a statistic represented by a multivariable function, the terms of the strong regularization variable are decomposed into variables used in the multivariable function, and then the regularization parameters are defined for the prediction formula after decomposition. For example, as shown in following formula (9), when the prediction model includes a variable $x_{y1}$ (main variable) indicating a value of inspection item 1 at the prediction reference point, a variable $x_{diff}$ (sub-variable and strong regularization variable) that indicates the difference between the value of inspection item 1 at the prediction reference point and the value of inspection item 1 at the time point immediately before the prediction reference point, and an arbitrary control variable, the prediction formula is rewritten as shown in following formula (10). In the formula (10), $x_{y2}$ is a variable indicating the value of inspection item 1 at the time point immediately before the prediction reference point. That is, $x_{diff} = x_{y1} - x_{y2}$. The terms for strong regularization in the formula (10) are the $x_{diff}$ term (second term) and the $x_{y2}$ term (third term). Therefore, the regularization parameter candidate setting unit 11 may generate a plurality of solution candidates having respectively different values set for at least the regularization parameter $\lambda_2$ corresponding to the $x_{diff}$ term (second term) and the regularization parameter $\lambda_3$ corresponding to the $x_{y2}$ term (third term).

$$y_1 = ax_{y1} + bx_{diff} + \ldots \quad + d \qquad \ldots \quad (9)$$

here,

$$ax_{y1} + bx_{diff}$$

$$= ax_{y1} + b(x_{y1} - x_{y2})$$

$$= (a + b)x_{y1} - bx_{y2}$$

$$= (a-c)x_{y1} + (b + c)x_{diff} + cx_{y2}$$

Therefore,

$$y_1 = (a-c)x_{y1} + (b + c)x_{diff} + cx_{y2} + \ldots \quad + d \qquad \ldots \quad (10)$$

Note that, the method of setting a plurality of solution candidates in the regularization parameter candidate setting unit 11 is not particularly limited. The regularization parameter candidate setting unit 11 can set solution candidates by the following method, for example.

[Setting method of solution candidate]

    (1) External condition setting
    (2) Grid search
    (3) Random setting

(1) External condition setting

The regularization parameter candidate setting unit 11 may set the search set (a plurality of solution candidates) explicitly given from the outside as it is.

(2) Grid search

Further, the regularization parameter candidate setting unit 11, for example, when the search range (for example, $0 \leq \lambda \leq 500$) of the regularization parameter and the grid width (for example, 100) are given from the outside as a condition of the regularization parameter, may use grid points ($\{0,100,200,300,400,500\}$) obtained by dividing the given search range into a grid as solution candidates for the normalization parameter corresponding to one strong regularization target term.

(3) Random setting

Further, the regularization parameter candidate setting unit 11, for example, when a distribution of the regularization parameter and the number of generations are given from the outside as conditions of the regularization parameter, may sample points for the number of generations specified from the given distribution, and use the obtained sampling value as a solution candidate of the normalization parameter corresponding to one strong regularization target term. The solution candidates are not limited to these, and may be those that the regularization parameter candidate setting unit 11 determined by calculation or based on external input.

The following is an example of a search set of regularization parameters in which a plurality of such solution candidates are set. Note that the following example is an example in which the strong regularization variable is $x_2$. In this example, v2-1 to v2-k corresponds to the value set in the regularization parameter $\lambda_2$ corresponding to the term of the strong regularization variable $x_2$.

- First solution candidate: $\lambda^{(1)} = \{\lambda_{1\,to\,M}\} = \{v1, v2\text{-}1, v3,..., vM\}$

- Second solution candidate: $\lambda^{(2)} = \{\lambda_{1\,to\,M}\} = \{v1, v2\text{-}2, v3,..., vM\}$

:

- Kth solution candidate: $\lambda^{(K)} = \{\lambda_{1\,to\,M}\} = \{v1, v2\text{-}k, v3,..., vM\}$

If there are a plurality of strong regularization variables, a plurality of $\lambda$ solutions (solution candidates) having at least different values expressed by the combination of regularization parameters $\lambda_{reg\_1}$ to $\lambda_{reg\_p}$ (p is the number of strong regularization variables) corresponding to each term of the strong regularization variable may be output. An example of such a plurality of $\lambda$ solution candidates is shown below. Note that the following example is an example in which the strong regularization variables are $x_2$ and $x_3$. v2-x (x is arbitrary) corresponds to the value of the regularization parameter $\lambda_2$ corresponding to the term of the strong regularization variable $x_2$. v3-x (x is arbitrary) corresponds to the value of the regularization parameter $\lambda_3$ corresponding to the term of the strong regularization variable $x_3$.

- First solution candidate: $\lambda^{(1)} = \{\lambda_{1\,to\,M}\} = \{v1, v2\text{-}1, v3\text{-}1,..., vM\}$

- Second solution candidate: $\lambda^{(2)} = \{\lambda_{1\,to\,M}\} = \{v1, v2\text{-}2, v3\text{-}1,..., vM\}$

:

- Kth solution candidate: $\lambda^{(K)} = \{\lambda_{1\,to\,M}\} = \{v1, v2\text{-}k, v3\text{-}1,..., vM\}$

:

- Lth solution candidate: $\lambda^{(L)} = \{\lambda_{1\,to\,M}\} = \{v1, v2\text{-}k, v3\text{-}h,..., vM\}$

Next, the model selection method by the model selection unit 13 will be described in more detail. The model selection unit 13, for example, as a result of performing prediction using predetermined verification data, may select a model having the smallest number of defective samples from among the models with the prediction accuracy (e.g., Root Mean Squared Error (RMSE) or correlation coefficient) equal to or more than a predetermined threshold. Examples of the threshold for the prediction accuracy include XX% or less of the prediction accuracy of the model with the highest prediction accuracy, a threshold (correlation coefficient 0.6, etc.) set by the user or domain expert, and the like.

In addition, the model selection unit 13, for example, as a result of performing prediction using predetermined verification data, may select a model having the highest prediction accuracy from among the models with the number of defective samples equal to or less than a predetermined threshold. Examples of the threshold for the number of defective samples include a threshold (1 digit etc.) set by the user or domain expert (an expert or the like in a technical field normally handling a predicted value, such as machine learning field and medical field), and XX% or less for the number of defective samples of the model having the smallest number of defective samples.

Further, the model selection unit 13 may obtain an accuracy score showing a positive correlation with respect to the obtained prediction accuracy and obtain a shape score showing a negative correlation with respect to the number of defective samples, and select a predetermined number of models in descending order of total score, which is represented by the sum of the two. In this way, it is possible to easily select a desired model by adjusting a positive weight used for calculating the accuracy score from the prediction accuracy and a negative weight used for calculating the shape score from the number of defective samples.

In addition, as a method for determining whether or not the sample is a defective sample, the following methods can be given.

- (1) Determine based on an error from an asymptotic line or a desired shape line in a predetermined confirmation period.

- (2) Determine based on a difference in a slope of a vector (hereinafter referred to as transition vector $\alpha$) that connects the predicted values between the two time points in the predetermined confirmation period.

- (3) Determine based on an angle (hereinafter referred to as transition angle β) formed by two straight lines represented by two adjacent transition vectors in a predetermined confirmation period.
- (4) Determine based on an amount of change in the predicted value in the predetermined confirmation period.

Here, the confirmation period is a period set for confirmation of a graph shape, and means a period in which at least two values including the actual value and the predicted value are given, and at least one prediction time point is included from the time point when the actual value is given. Note that the same number of time points and number of prediction time points as those in the evaluation target period may be used in the confirmation period.

For example, in the above method (1), when a predicted value at each time point included in the confirmation period is obtained for a combination of explanatory variables, an amount of error between the predicted value and a curve (approximate curve) obtained by curve fitting to the transition of the predicted value, that is, the sum of differences between the value at each time point on the approximate curve and each predicted value is obtained as an asymptotic score. Then, if the asymptotic score is equal to or more than the predetermined threshold, the combination may be determined to be a defective sample. Fig. 6 is an explanatory diagram showing an example of curve fitting in progress prediction. Fig. 6A is an example in which a small asymptotic score is calculated as a result of curve fitting, and Fig. 6B is an example in which a large asymptotic score is calculated as a result of curve fitting. Note that the method of curve fitting is not particularly limited, but for example, it may be a method for obtaining a curve that best fits experimentally obtained data (e.g., transition of experimental values) or predetermined constraint conditions defined according to a desired graph shape.

Further, in the above method of (1), for example, the model selection unit 13, when having obtained a predicted value at each time point included in the confirmation period for a combination of explanatory variables, may calculate an invalidity score, which is an index indicating an invalidity of the transition of the predicted value (more specifically, it is series data including data that indicates the obtained predicted value, and is series data including three or more pieces of data that indicate the value of the prediction target item in association with time) and an invalidity score based on the error between the curve model obtained by fitting the series data to a predetermined function form and the series data, and determine the combination to be a defective sample, if the calculated invalidity score is equal to or smaller than a predetermined threshold. In addition, the invalidity score is not only used to determine whether the sample is a defective sample or not, but can be used as it is as an evaluation index regarding a graph shape by defining the index showing a negative correlation with the calculated invalidity score as a shape score. The details of the calculation method of the invalidity score will be described later.

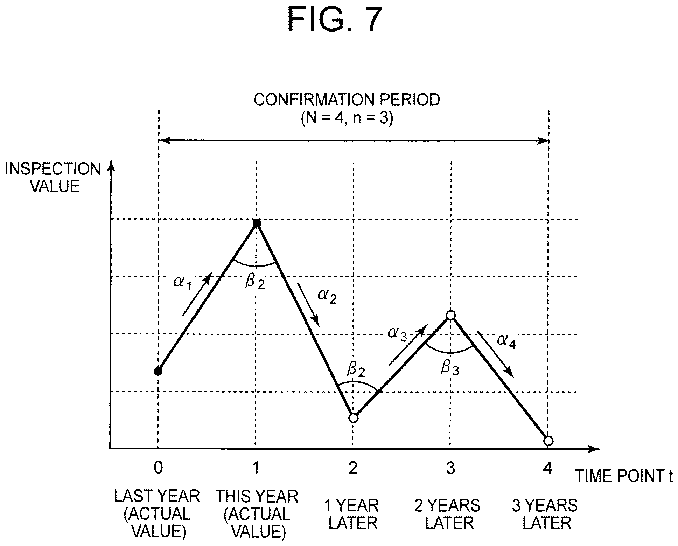

Further, Fig. 7 is an explanatory diagram showing an example of transition vectors and transition angles in the progress prediction. For example, in the above method (2), the slopes $d_1$ to $d_4$ may be obtained for the transition vectors $\alpha_1$ to $\alpha_4$ as shown in Fig. 7, and wscore shown below may be obtained from each obtained slope d. In formula (11), $|d_i-d_{i+1}|$ represents a difference between the slope $d_i$ of a vector (transition vector $\alpha_i$) from the value of the prediction target item at the time point $t^{(i-1)}$ to the value of the prediction target item at the time point $t^{(i)}$ and the slope $d_{i+1}$ of a vector (transition vector $\alpha_{i+1}$) from the value of the prediction target item at time point $t^{(i)}$ to the value of the prediction target item at time point $t^{(i+1)}$.

$$\text{wscore} = |d_1\text{-}d_2|+|d_2\text{-}d_3|+...\quad+|d_{n-1}\text{-}d_n|$$

$$...\quad(11)$$

The wscore of this example shows a small value when the amount of change in the slope is small, and a large value when the amount of change in the slope is large. Therefore, the model selection unit 13 may determine that the shape is defective (a sample that cannot be interpreted) if the wscore is equal to or larger than a predetermined threshold.

Further, for example, in the above method (3), the transition angles $\beta_1$ to $\beta_3$ may be obtained from the transition vectors $\alpha_1$ to $\alpha_4$ as shown in Fig. 7, and the vscore shown below may be obtained from each obtained transition angle β [rad]. In formula (12), $0 \leq \beta_i \leq \pi$. Here, the transition angle $\beta_i$ is an angle formed by two straight lines represented by the two transition vectors $\alpha_i$ and $\alpha_{i+1}$.

$$\text{vscore} = (1/\pi)(\pi\text{-}\beta_1) +...\quad + (1/\pi)(\pi\text{-}\beta_{n-1})$$

$$...\quad(12)$$

The vscore in this example shows a larger value as the transition angle becomes smaller. Therefore, the model selection unit 13 may determine that the shape is defective (the sample that cannot be interpreted) if the vscore is equal to or larger than a predetermined threshold, for example.

Further, for example, in the above method (4), when two predicted values ($y_1$, $y_2$) are given, the ascore is obtained as follows, and if the obtained ascore is equal to or more than a threshold, the shape may be determined to be defective.

$$\text{ascore} = |y_1 - y_2| - a \text{ (when } |y_1 - y_2| \geq a)$$

$$\text{ascore} = 0 \text{ (other times)}$$

Note that when three or more predicted values are given, the sum of absolute values of differences between adjacent time points may be used instead of $|y_1 - y_2|$.

The model selection unit 13 may use the wscore, vscore, or ascore instead of or in addition to the number of defective samples when evaluating the model.

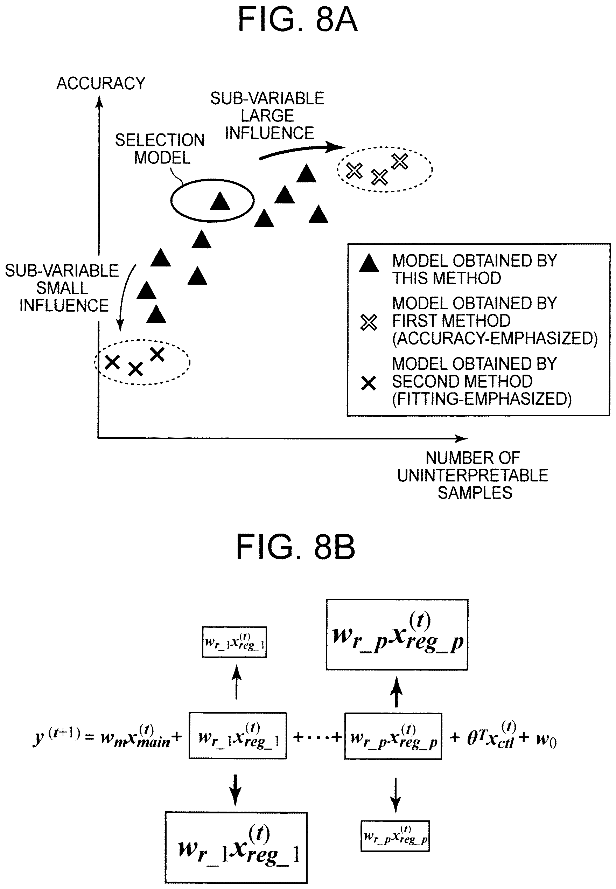

Fig. 8 is a conceptual diagram showing the effect of adjusting the regularization parameter of the present exemplary embodiment. For example, when the model is learned by emphasizing the prediction accuracy without considering the graph shape in the first method, that is, the progress prediction, the prediction accuracy is high, but the number of defective samples increases, as shown by a white cross mark in Fig. 8A. In the second method, that is, when the model is learned by emphasizing the fitting to the desired graph shape, the number of defective samples is small, but the prediction accuracy is low, as shown by a black cross mark in Fig. 8A. On the other hand, according to the method of the present exemplary embodiment, by individually adjusting the regularization parameter corresponding to the term of the strong regularization variable and verifying the result, for example, it is possible to select a model with a good balance between the prediction accuracy and the number of defective samples, such as a model surrounded by the solid circle in the figure.

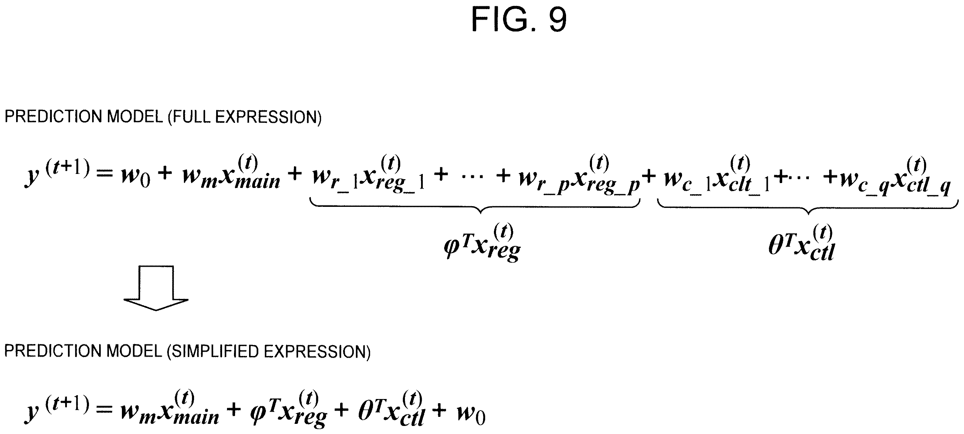

Further, Fig. 8B is an explanatory diagram schematically showing a method of adjusting the balance between the prediction accuracy and the number of defective samples. In Fig. 8B, a part of the prediction formula of the prediction model is simplified and shown by the method shown in Fig. 9. In the prediction formula shown in Fig. 9, $x_{main}$ is a main variable, $w_m$ is a main coefficient, $x_{reg}$ in bold is a column vector of sub-variables that are strong regularization variables, and $\varphi^T$ in bold is a row vector of coefficient of a sub-variable that is a strong regularization variable, $x_{ct1}$ in bold indicates a column vector of control variable, $\theta^T$ in bold indicates a row vector of coefficient of control variable, and wo indicates an intercept. As shown in Fig. 9, the prediction model may be a prediction model in which only (1) a main variable, (2) a sub-variable that is a strong regularization variable, and (3) a control variable are explanatory variables.

As shown in Fig. 8B, since the magnitude of the coefficient $w_{reg}$ in each term of the sub-variable can be individually adjusted by changing the value of the regularization parameter corresponding to each term of the strong regularization variable ($x_{reg\_1}$ to $x_{reg\_p}$ in the example in the figure), the effect of each sub-variable on the predicted value can be adjusted. For example, if the value of the regularization parameter $\lambda_{reg\_1}$ corresponding to the term of the variable $x_{reg\_1}$ that is one of the strong regularization variables is made relatively large with respect to the value of the regularization parameter corresponding to the other terms, the magnitude of the coefficient $w_{r\_1}$ of the term of $x_{reg\_1}$ can be reduced. As a result, the effect of the variable $x_{reg\_1}$ on the predicted value can be reduced. In Fig. 8B, the magnitude of the effect of the strong regularization variable on the predicted value is schematically shown by using the size of the term of the strong regularization variable (see the square frame in the figure).

As described above, according to the present exemplary embodiment, it is possible to generate a model with a good balance between the prediction accuracy and the number of defective samples without limiting the explanatory variables used in the prediction model. Therefore, for example, even when the progress prediction of a certain inspection value is performed in a health simulation or the like by keeping the items related to lifestyle habits constant, it is possible to reduce the possibility of presenting an uninterpretable prediction result while maintaining the prediction accuracy.

In the above description, an example is shown in which model learning to model selection is performed by a single search for regularization parameters (setting of solution candidates), but it is also possible to further include a higher-order module that controls the three modules of the regularization parameter candidate setting unit 11, the model learning unit 12, and the model selection unit 13, so that a better range can be searched sequentially.

In the example shown in Fig. 3, the model selection unit 13 selects a model to be used for prediction from among a plurality of models having different values of the regularization parameter corresponding to the strong regularization variable at the time of model generation. However, the selection of model can be performed also in the prediction phase, for example.

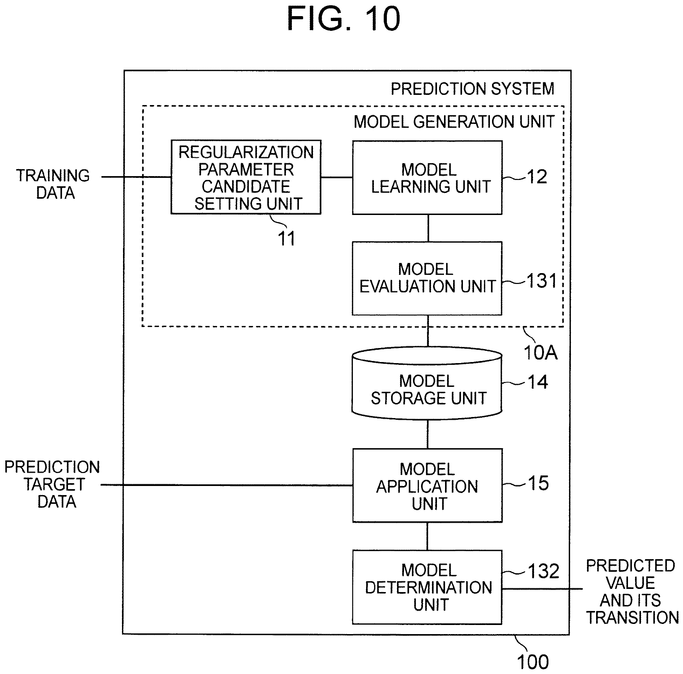

Fig. 10 is a block diagram showing another configuration example of the prediction system of the present exemplary embodiment. The prediction system shown in Fig. 10 includes a model generation unit 10A including a regularization parameter candidate setting unit 11, a model learning unit 12, and a model evaluation unit 131, a model storage unit 14, a model application unit 15, and a model determination unit 132.

The model evaluation unit 131 evaluates at least a prediction accuracy for each of a plurality of prediction models (learned models having mutually different values of the regularization parameter corresponding to the strong regularization variable) learned by the model learning unit 12, the prediction model corresponding to each solution candidate included in the search set in which the regularization parameter is determined by the regularization parameter candidate setting unit 11. The model evaluation unit 131 may apply, for example, predetermined verification data to each of the plurality of learned prediction models, and evaluate the prediction accuracy based on the difference between the obtained predicted value and the target value. Note that the model evaluation unit 131 may further obtain the number of defective samples, wscore, vscore, shape score, etc. when having performed the progress prediction as an index related to the graph shape (the graph shape indicating the transition of the predicted value at the time of the progress prediction), and evaluate the graph shape based on the obtained index. The evaluation result of the model evaluation unit 131 is stored in the model storage unit 14.

It should be noted that such a model evaluation unit 131 can also be implemented by utilizing a part of the function (function of evaluating a model) of the model selection unit 13 of the first exemplary embodiment. In that case, the function (model evaluation function) may store, in the model storage unit 14, the evaluation index (prediction accuracy, accuracy score, number of defective samples, shape score, total score, wscore, vscore, etc.) of each model obtained based on the verification data, together with the model information.

When the prediction target data is input, the model application unit 15 of the present example reads out a plurality of learned prediction models stored in the model storage unit 14, applies the prediction target data to each read prediction model, and obtains the predicted value at each prediction time point included in the evaluation target period for each model.

The model determination unit 132, based on the evaluation result of each model stored in the model storage unit 14 and the predicted value at each prediction time point included in the evaluation target period for each model obtained by the model application unit 15, obtains the final predicted value by selecting (determining) one model that obtains the final predicted value from among the plurality of models that have obtained the predicted values. For example, the model determination unit 132, after evaluating the graph shape indicated by the prediction result (predicted value at each prediction time point) obtained from the current prediction target data, may determine a model based on the evaluation result (current evaluation result) and the evaluation result (past evaluation result) of each model stored in the model storage unit 14.

Similarly to the above, the evaluation of the graph shape can be performed by determining whether or not the sample is a defective sample, and obtaining the wscore, vscore, shape score, and the like.

Further, as the model determination method in the model determination unit 132, the model selection method by the model selection unit 13 described above can be used. At this time, the model determination unit 132 uses the evaluation result stored in the model storage unit 14 for the evaluation index regarding the prediction accuracy such as the prediction accuracy and the accuracy score. On the other hand, the model determination unit 132, for the number of defective samples, and the evaluation index regarding the graph shape such as the wscore, vscore, and shape score, may use only the current evaluation result, or the evaluation result obtained by putting together the current evaluation result and the past evaluation result (the evaluation result obtained by adding the current evaluation result to the evaluation result stored in the model storage unit 14).

For example, when only the current evaluation result is used as the evaluation index regarding the graph shape, a model most suitable for the current sample, that is, for the current prediction target data (for example, a model that outputs the most accurate predicted value while excluding the predicted value indicating an invalid change) can be selected. Further, for example, when the evaluation result obtained by putting together the current evaluation result and the past evaluation result is used as an evaluation index regarding the graph shape, a model most suitable for many samples not limited to the current sample (for example, a model with a good balance between the prediction accuracy and the number of defective samples) can be selected.

When the model determination unit 132 determines one model, the predicted value at each prediction time point included in the evaluation target period obtained from the model is output as the final predicted value.

Note that other points may be similar to those of the prediction system shown in Fig. 3. In this way, the same effect can be obtained even when the model is selected (determined) at the time of prediction.

Further, in the above description, an example in which the prediction system 100 includes the model generation unit 10 and the model generation unit 10A has been shown, but, for example, it is also possible to store the prediction model as described above in the model storage unit 14 in advance. In that case, the model generation unit 10 and the model generation unit 10A can be omitted.

Second exemplary embodiment

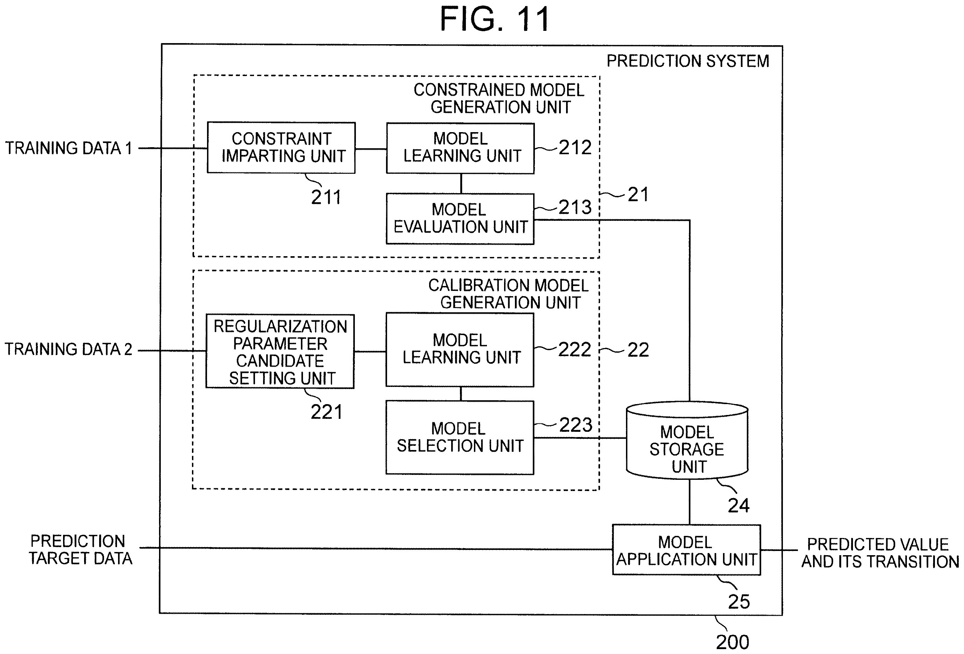

Next, a second exemplary embodiment of the present invention will be described. Fig. 11 is a block diagram showing a configuration example of a prediction system of the second exemplary embodiment. A prediction system 200 shown in Fig. 11 includes a constrained model generation unit 21, a calibration model generation unit 22, a model storage unit 24, and a model application unit 25.

The prediction system 200 shown in Fig. 11 is different from the first exemplary embodiment shown in Fig. 3 in that the constrained model generation unit 21 is added, and the model generation unit 10, the model storage unit 14, and the model application unit 15 are different from the calibration model generation unit 22, the model storage unit 24, and the model application unit 25, respectively.

The constrained model generation unit 21 is a processing unit that generates a prediction model using the second method, that is, a method emphasizing fitting to a desired graph shape, and includes a constraint imparting unit 211, a model learning unit 212, and a model evaluation unit 213. Hereinafter, a prediction model generated by the constrained model generation unit 21 will be referred to as a constrained prediction model.

The constrained prediction model is not particularly limited as long as it is a prediction model that does not use two or more non-control variables such as a main variable and sub-variables for explanatory variables of the prediction model. The constrained prediction model may be a linear model or a piecewise linear model on which in addition to the constraint that two or more non-control variables are not used, for example, a constraint that in the prediction formula, a coefficient of an explanatory variable corresponding to one non-control variable (for example, a main variable) falls within a range of value designated in advance is imposed. Such a constrained prediction model can be generated by limiting the explanatory variables used in the prediction formula to one non-control variable (for example, main variable) and one or more control variables, and then performing model learning by imposing the constraint so that the coefficient of the non-control variable falls within a range of value designated in advance.

In addition, when the prediction model is a piecewise linear model including a section definition, a constraint that only the control variable is used for the section definition may be further imposed. This makes it possible to prevent the prediction formulas from switching in the piecewise linear model when the control variable is constant.

By adding such a constraint, it is possible to prevent a predicted value indicating an invalid transition from being output. In addition, the range of value of the coefficient of the non-control variable may be defined according to a predetermined type as a valid transition type in the value of the prediction target item.

Due to the above constraints, the prediction formula of the prediction model can be expressed as, for example, formula (13). Here, $x_{main}$ represents the main variable, a represents the coefficient of the main variable, $x_q$ represents the column vector of the control variable, $\theta^T$ represents the row vector of the coefficient of the control variable, and b represents the intercept. Although the main variable is used as one non-control variable in the formula (13), one non-control variable may be other than the main variable.

[Math 4]

$$y^{(t+1)} = ax_{main}^{(t)} + \theta^T x_{ctl}^{(t)} + b \quad \cdot \cdot \cdot \quad (13)$$

$$\text{where,} \quad x_{main}^{(t+1)} \leftarrow y^{(t+1)}$$

Then, by solving a recurrence formula of the prediction formula (13) with t = 0, a general formula indicating the value of the prediction item at the n-th prediction time point can be derived as formula (14).

[Math 5]

$$y^{(t+n)} = a^n \left( x_{main}^{(t)} - \frac{\theta^T x_{ctl}^{(t)} + b}{1-a} \right) + \frac{\theta^T x_{ctl}^{(t)} + b}{1-a} \quad \cdot \cdot \cdot \quad (14)$$

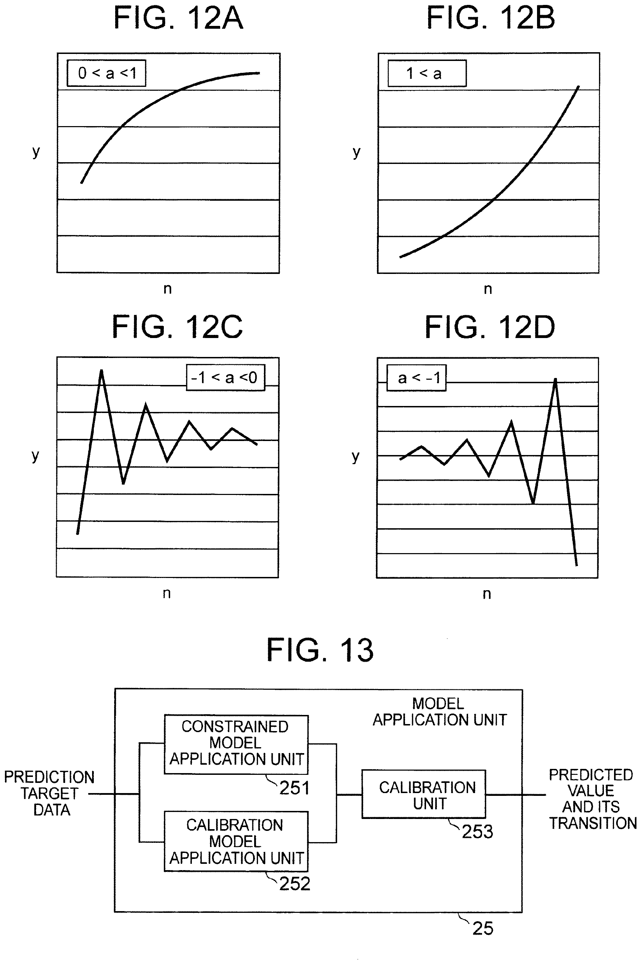

After limiting the prediction formula used in the prediction model to the prediction formula expressed by such a recurrence formula, by limiting the range of value of the coefficient (a described above) of the non-control variable to a predetermined range, it is possible to prevent the prediction formula from outputting a predicted value that shows an invalid change under the condition that the control variable is constant. As an example, under such a constraint, for example, if the range of value of the coefficient a of the non-control variable is limited to 0 < a < 1, then the graph shape can be limited to an upward asymptotic shape as shown in Fig. 12A. Further, for example, if it is limited to 1 < a, it can be limited to a graph shape that gradually approaches downward as shown in Fig. 12B. Further, for example, if it is limited to -1 < a < 0, it can be limited to a graph shape that gradually converges as shown in Fig. 12C. Further, for example, if it is limited to -1 > a, it can be limited to a graph shape that gradually diverges as shown in Fig. 12D. It should be noted that these valid graph shapes may include a case where y is a constant value (a = 0), a simple increase (a = 1), or a simple decrease (a = -1), as required.

The range of value of the coefficient a of the non-control variable is not limited to the above, and may be any value that matches a predetermined valid transition type of the predicted value.

The constraint imparting unit 211 imparts the above-described constraint to the model learning unit 212 in the subsequent stage.

The model learning unit 212 learns a model using given first training data under the constraint given by the constraint imparting unit 211. Here, the first training data (training data 1 in the figure) given to the model learning unit 212 may be a data set in which the value of a prediction target item which is the target variable in the constrained prediction model, and a plurality of variables (more specifically, a combination of the plurality of variables) that can be correlated with the value of the prediction target item and are explanatory variables in the constrained prediction model are associated with each other. For example, the first training data may be data obtained by removing the sub-variables from among the plurality of variables that are the explanatory variables in the training data of the first exemplary embodiment. The constrained model generation unit 21, for example, similarly to the first exemplary embodiment, after inputting the training data including the main variable, the sub-variable and the control variable to the explanatory variables, may generate training data to be given to the model learning unit 212 by the constraint imparting unit 211 performing data processing (for example, such as setting the value to zero, and deleting from the data structure itself) of excluding the explanatory variable corresponding to the sub-variable from among the input training data.

The model evaluation unit 213 evaluates the prediction accuracy of the constrained model learned by the model learning unit 212. The model evaluation unit 213 may, for example, apply predetermined verification data to each of the learned constrained prediction models, and evaluate the prediction accuracy based on the difference between the obtained predicted value and the target value. At this time, the model evaluation unit 213 calculates a value (for example, residual, log residual, residual sum of squares, etc.) used for calibration of the predicted value obtained from the prediction model (learned constrained prediction model) in the prediction phase. The value used for calibration (hereinafter referred to as a calibration value) is not limited to the above example as long as it can be calculated from the target value (value of the target variable) indicated by the given first training data, and a predicted value obtained by applying the training data to the learned constrained prediction model. In the following, the calibration value calculated based on the target value indicated by the training data and the predicted value actually acquired from the combination of the explanatory variables associated with the target value may be referred to as an actual calibration value.

The learned constrained model is stored in the model storage unit 24. At this time, for the purpose of learning the calibration model described later, information that associates the actual calibration value obtained from the training data with the combination of the explanatory variables having obtained the actual calibration value may also be stored together.

Further, the calibration model generation unit 22, using the method shown in the first exemplary embodiment, generates a plurality of learned prediction models corresponding to each of a plurality of solution candidates having at least different values for the regularization parameter that affects the term of the predetermined strong regularization variable. However, the calibration model generation unit 22 of the present exemplary embodiment generates, using the method shown in the first exemplary embodiment, the prediction model for predicting the calibration value for the predicted value output by the conditional prediction model for arbitrary prediction target data. Hereinafter, the prediction model generated by the calibration model generation unit 22 will be referred to as a calibration model.

That is, the calibration model may be any prediction model having a variable indicating a calibration value with respect to the predicted value output from the conditional prediction model for arbitrary prediction target data as a target variable. The explanatory variable is not particularly limited, but may be a variable indicating a value that can be acquired at the prediction reference point and may be a variable that can be correlated with the calibration value. Note that the explanatory variables of the calibration model may be a plurality of variables that can be correlated with the value of the prediction target item at the prediction time point one ahead of the prediction reference point, as in the first exemplary embodiment. In other words, it may be one in which one or more non-control variables are further added to the plurality of variables that are the explanatory variables in the constrained model.

The regularization parameter candidate setting unit 221, the model learning unit 222, and the model selection unit 223 in the calibration model generation unit 22 may be basically the same as the regularization parameter candidate setting unit 11, the model learning unit 12, and the model selection unit 13 of the first exemplary embodiment except that the prediction model to be generated is the calibration model, that is, the target variables are different.

That is, the regularization parameter candidate setting unit 221, when the second training data (training data 2 in the figure) is input, outputs a search set in which a plurality of solution candidates are set, the solution candidates having at least different values of the regularization parameter that affects the term of the strong regularization variable among the regularization parameters that are parameters used for regularization of the calibration model.

The model learning unit 222 uses the second training data to learn the calibration model corresponding to each solution candidate included in the search set of the regularization parameter output from the regularization parameter candidate setting unit 221.

The model selection unit 223 selects a prediction model having high prediction accuracy and a small number of defective samples from among the calibration models respectively corresponding to the plurality of solution candidates of the regularization parameter learned by the model learning unit 222, and stores it in the model storage unit 24.

However, in the calibration model generation unit 22 (regularization parameter candidate setting unit 221, model learning unit 222 and model selection unit 223) of the present exemplary embodiment, the predicted value and the target value of the prediction model are the predicted value (calibration value) and the target value (actual calibration value) of the calibration model. As the target value (actual calibration value) of the calibration model, for example, one included in the input second training data may be used. That is, if the second training data is a data set in which the combination of the explanatory variables and the actual calibration value in the combination of the explanatory variables are associated with each other, the actual calibration value included in the second training data may be used. Incidentally, if the second training data is a data set in which the combination of the explanatory variables and the value (the target value of the constrained model) of the actual prediction item in the combination of the explanatory variables are associated with each other, the actual calibration value may be calculated from the predicted value obtained by applying the combination (however, non-control variables other than one non-control variable are excluded) to the constrained model and the target value.

When the prediction target data is input, the model application unit 25 uses the constrained model and the calibration model stored in the model storage unit 24 to perform progress prediction. Specifically, the model application unit 25 may apply the prediction target data to the constrained model and the calibration model stored in the model storage unit 14 to obtain the value of the prediction target item at each prediction time point included in the evaluation target period. The prediction target data input to the model application unit 25 may be a combination of a plurality of arbitrary variables including at least the explanatory variable of the constrained model and the explanatory variable of the calibration model.

Fig. 13 is a block diagram showing a configuration example of the model application unit 25. As shown in Fig. 13, the model application unit 25 includes a constrained model application unit 251, a calibration model application unit 252, and a calibration unit 253.

When the prediction target data is input, the constrained model application unit 251 uses the constrained model stored in the model storage unit 24 to perform progress prediction of the value of the prediction target item. Specifically, the constrained model application unit 251 may read the constrained model stored in the model storage unit 24, apply prediction target data (however, a non-control variable other than one non-control variable (for example, (sub-variable) is excluded) to the constrained model, and obtain a predicted value (value of the prediction target item. Hereinafter, called a first predicted value) at each prediction time point included in the evaluation target period. At this time, the constrained model application unit 251 performs a process of excluding the non-control variables other than one non-control variable from the prediction target data as required.

When the prediction target data is input, the calibration model application unit 252 uses the calibration model stored in the model storage unit 24 to acquire the calibration value of the first predicted value at each prediction time point obtained from the constrained model. The calibration model application unit 252, for example, may read the calibration model stored in the model storage unit 24, apply the prediction target data to the calibration model, and obtain the calibration value of the first predicted value at each prediction time point included in the evaluation target period.

The calibration unit 253 calibrates the first predicted value at each prediction time point included in the evaluation target period obtained by the constrained model application unit 251, based on the calibration value for the value obtained by the calibration model application unit 252. Then, the first predicted value at each prediction time point after calibration is output as the final predicted value, that is, the value of the prediction target item at each prediction time point.

Next, the operation of the prediction system of the present exemplary embodiment will be described. The operation of the prediction system of the present exemplary embodiment is also roughly divided into a model learning phase for learning a prediction model (constrained model and calibration model) using training data, and a prediction phase for performing progress prediction using the learned prediction model.

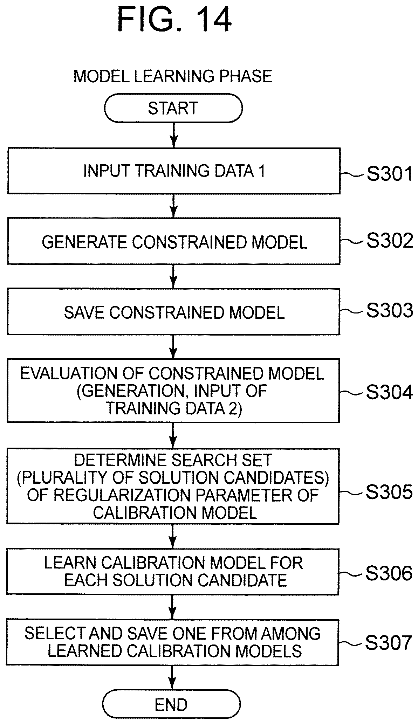

First, the model learning phase will be described. The model learning phase is performed at least once before the prediction phase. Fig. 14 depicts a flowchart showing an operation example of the model learning phase of the prediction system of the present exemplary embodiment.

In the example shown in Fig. 14, first, the first training data is input to the prediction system (step S301). In this example, as the first training data, a data set indicating a combination of variables including the value (target value of the constrained model) of the prediction target item at a certain time point t', the main variable when the time point (t'-1) immediately before the time point t' is a prediction reference point, and one or more control variables is input. The value of the control variable is arbitrary. Note that the first training data may be a data set indicating a combination of variables that further include sub-variables when the time point (t'-1) is the prediction reference point. In that case, the second training data can be generated from the first training data.

In step S301, information on constraints given to the constrained model may be accepted together with the training data.

Then, the constrained model generation unit 21, based on the input first training data, generates a constrained model on which the above constraint is imposed (step S302), and saves it in the model storage unit 24 (step S303).

When the constrained model is stored in the model storage unit 24, the calibration model generation unit 22 evaluates the constrained model, calculates an actual calibration value that is a target value of the calibration model to generate the second training data, and then inputs the second training data (step S304). In this example, as the second training data, a data set indicating a combination of variables including the calibration value (a target value of the calibration model) of the value of the prediction target item at a certain time point t', a main variable and one or more sub-variables when the time point (t'-1) immediately before the time point t' is the prediction reference point, and an arbitrary control variable is input.

In step S304, the regularization parameter conditions and the like may be accepted together with the second training data.

Next, the regularization parameter candidate setting unit 221 outputs a search set in which a plurality of solution candidates are set, the solution candidates having different values of the regularization parameter that affects at least the term of the strong regularization variable among the explanatory variables of the calibration model. (step S305).

Next, the model learning unit 222 uses the second training data to respectively learn the calibration model corresponding to each solution candidate of the regularization parameter output from the regularization parameter candidate setting unit 221 (step S306).

Next, the model selection unit 223 selects a calibration model having high prediction accuracy and a small number of defective samples from among the plurality of learned calibration models, and stores it in the model storage unit 24 (step S307).

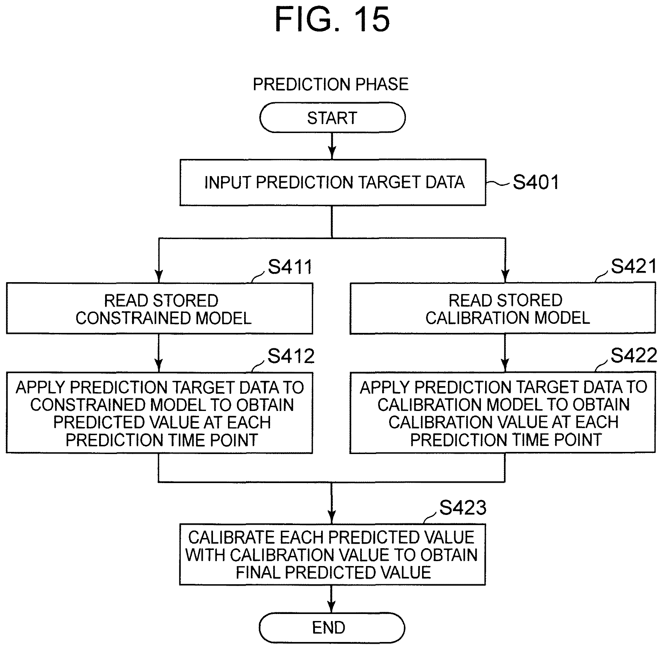

Next, the prediction phase will be described. Fig. 15 is a flowchart showing an operation example of the prediction phase of the prediction system of the present exemplary embodiment. The prediction phase may be performed, for example, every time a progress prediction is requested.

In the example shown in Fig. 15, first, prediction target data is input to the prediction system (step S401). The prediction target data input here may be data indicating an arbitrary combination of a plurality of variables including variables that are explanatory variables in the constrained model and the calibration model stored in the model storage unit 24. In this example, data indicating a combination of variables including the main variable and sub-variables when the time point (first prediction reference point) immediately before the first prediction time point (first prediction time point) in the evaluation target period of the current progress prediction is the prediction reference point, and one or more control variables is input. Also in the present exemplary embodiment, the value and the like of the control variable used for the prediction at each prediction time point can be specified as the prediction condition.

When the prediction target data is input, the model application unit 25 uses the constrained model and the calibration model stored in the model storage unit 24 to perform progress prediction (step S411 to step S412, step S421 to steps S422, and step S423).

When the prediction target data is input, for example, the constrained model application unit 251 of the model application unit 25 reads the constrained model from the model storage unit 24 (step S411), applies the prediction target data (however, the sub-variables are excluded) to the read constrained model, and obtains a predicted value (first predicted value) at each prediction time point included in the evaluation target period (step S412).

Further, when the prediction target data is input, for example, the calibration model application unit 252 of the model application unit 25 reads the calibration model from the model storage unit 24 (step S421), applies the prediction target data to the read calibration model, and obtains a predicted value (calibration value of the first predicted value) at each prediction time point included in the evaluation target period (step S422).

Then, the calibration unit 253 of the model application unit 25 calibrates the first predicted value at each prediction time point using the calibration value to obtain a final predicted value at each prediction time point (step S423).

As described above, according to the present exemplary embodiment, since it is possible to calibrate the predicted value obtained by the constrained model limited so as to output the predicted value having the predetermined graph shape, with the calibration value by the calibration model in consideration of both the prediction accuracy and the shape accuracy, it is possible to obtain the same effect as that of the first exemplary embodiment. That is, it is possible to reduce the possibility of presenting an uninterpretable prediction result while maintaining the prediction accuracy.

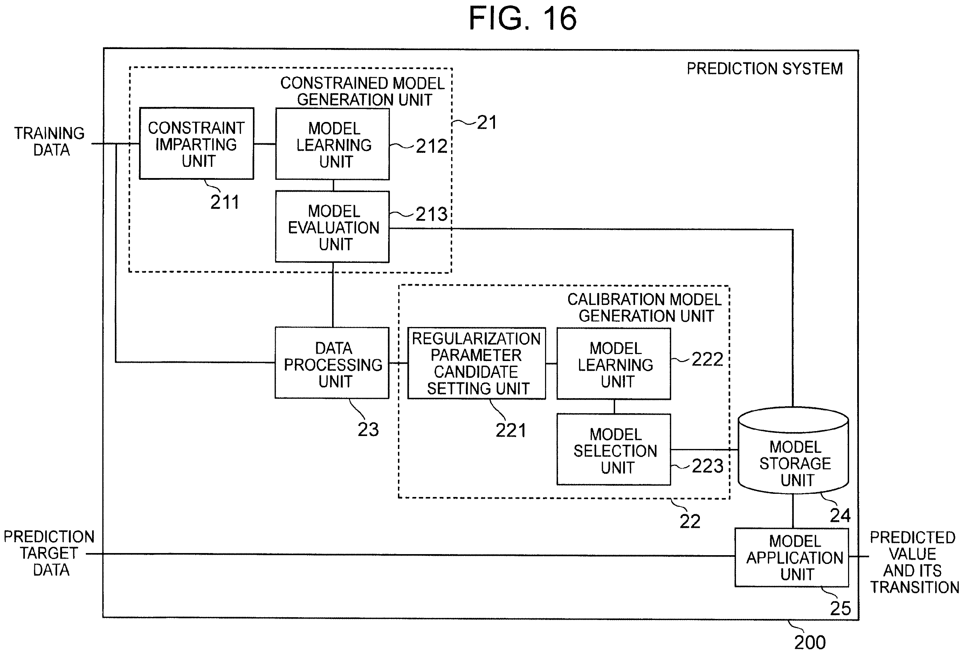

Note that Fig. 16 is a block diagram showing another configuration example of the prediction system of the present exemplary embodiment. In the example shown in Fig. 16, to the prediction system 200, a data processing unit 23 that performs a process of generating second training data from training data that includes sub-variables as explanatory variables, as shown in step S304 described above.

Further, although not shown in the drawings, also in the present exemplary embodiment, a part of the function (model selection processing) by the model selection unit 223 may be performed in the prediction phase. In that case, a model determination unit may be further provided after the model application unit 25. Note that the model determination method by the model determination unit may be basically the same as that of the model determination unit 132 of the first exemplary embodiment.

Also in the present exemplary embodiment, it is also possible to previously store the constrained model and the calibration model as described above in the model storage unit 24. In that case, the constrained model generation unit 21 and the calibration model generation unit 22 can be omitted.

In each of the above-described exemplary embodiments, an example in which a model is selected based on the evaluation result regarding the prediction accuracy and the evaluation result regarding the graph shape or the number of defective samples is shown. However, a timing to perform these evaluations is not limited to the time for model selection. For example, after the model selection, it is also possible to further perform these evaluations in the shipping determination or the like of the prediction result of the progress prediction performed using the model.

In that case, for example, a shipping determination unit (not shown) may input transition of the predicted value obtained from the model to be shipped or transition of the predicted value to be shipped (more specifically, series data including data indicating the obtained predicted value, the series data including two or more pieces of data indicating the value of the prediction target item in association with time, perform evaluation regarding the prediction accuracy as described above or evaluation regarding the graph shape or the number of defective samples on the input series data, and make a shipping determination of the model that has obtained the predicted value included in the series data or the predicted value included in the series data, based on those evaluation results.

The shipping determination may be made, for example, by determining whether or not the total score represented by the sum of the accuracy score indicating a positive correlation with the obtained prediction accuracy and the shape score indicating a negative correlation with the number of defective samples is equal to or more than a predetermined threshold. In shipping determination, only the evaluation regarding the graph shape or the number of defective samples may be performed, and the availability of shipping may be determined based on the obtained evaluation result.

Further, the shipping determination unit, for example, when the predicted values for a plurality of samples are obtained from one prediction model, if all the series data including the predicted value in each sample can be shipped, may determine that the shipment of the predicted values included in them is OK, and if other than that, may perform predetermined alert processing. Further, for example, when the series data is obtained from each of a plurality of prediction target items, the shipping determination unit may evaluate them individually or may collectively evaluate them (collective evaluation). As an example, the shipping determination unit may collectively evaluate, for each shipping unit that is a unit in which the predicted value or the prediction model is shipped, the series data including the predicted value in the shipping unit.

As the alert processing, for example, it may be output to a predetermined server or a display device together with the series data of the prediction target item that is determined to be unshippable, and a manual shipment availability determination may be requested. Further, the result of manual shipment availability determination may be accepted.

It should be noted that it is also possible to provide a shipping determination device that semi-automatically performs input and shipping determination of such series data independently of a device or a system that performs prediction.

Third exemplary embodiment

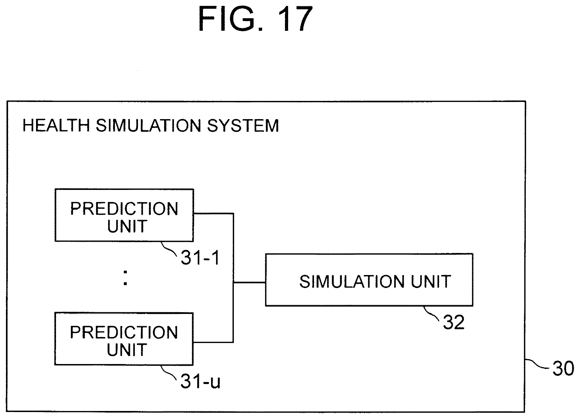

Next, a health simulation system will be described as an example of using the prediction system according to each of the above-described exemplary embodiments. Fig. 17 is a block diagram showing a configuration example of the health simulation system of the third exemplary embodiment. A health simulation system 30 of the present exemplary embodiment includes prediction units 31 (31-1 to 31-u) corresponding to the number u of inspection items used for the health simulation, and a simulation unit 32.

Each of the prediction units 31-1 to 31-u is realized by any of the above prediction systems. The prediction unit 31-1 to 31-u specifically predicts and outputs, for one inspection item i (for example, any of the inspection items 1 to u related to lifestyle-related diseases) that is predetermined as a prediction target item, a value of the prediction item at each prediction time point included in the specified evaluation target period, based on the input prediction target data.

Incidentally, each prediction unit 31 preliminarily stores, for the target value $y_i^{(t')}$ of the prediction target item at a certain time point t' as a target variable, at least one prediction model (or a pair of a constrained model and a calibration model) learned using training data including at least, as explanatory variables, a variable $x_{main}^{(t'-1)}$ corresponding to the value of the prediction target item at the time point t'-1, a value $\{x_{subj}^{(t'-1)}\}$ (where j = 1 to p) of one or more arbitrary variables other than the $x_{main}^{(t'-1)}$ indicating a value that can be acquired at the time point t'-1, and a value $\{x_{clt\_i}^{(t'-1)}\}$ (where i = 1 to q) of one or more inquiry items related to lifestyle habits indicating a value that can be acquired at the time point t'-1.

Here, $x_{main}^{(t'-1)}$ corresponds to the main variable, $x_{sub\_j}^{(t'-1)}$ corresponds to the sub-variable, and $x_{clt\_i}^{(t'-1)}$ corresponds to the control variable. Here, $x_{sub\_j}^{(t'-1)}$ may be, for example, a variable $x_{main}^{(t'-2)}$ indicating a value of the prediction target item at the time point t'-2 or a variable $x_{diff}^{(t'-1)}$ (= $x_{main}^{(t'-1)}$ - $x_{main}^{(t'-2)}$) indicating the difference between a value of the prediction target item at the time point t'-1 and a value of the prediction target item at the time point t'-2. The sub variables are not limited to these variables. For example, it may be a statistical value (including a difference) calculated from the value of the prediction target item at the time point t'-1 and the value of another inspection item at the time point t'-1.

For example, $x_{main}^{(t'-1)}$ in the prediction unit 31-i may be an explanatory variable corresponding to $x_1^{(t)}$ in the above formula (3). Also, for example, $x_{sub\_j}^{(t'-1)}$ may be an explanatory variable corresponding to $x_{i'}^{(t)}$ (where i' = 1 to u, but excluding i) in the above formula (3).

Note that $x_{clt\_i}^{(t'-1)}$ is not particularly limited as long as it is a value that can be acquired and can be controlled at the time point t'-1. For example, $x_{clt\_i}^{(t'-1)}$ may be a value of the inquiry item related to lifestyle habits at the time point t' corresponding to the first prediction time point (first prediction time point) seen from the time point t'-1. In that case, it means that a future value is set for the inquiry item related to lifestyle habits at the time point t'-1.

The simulation unit 32 uses the prediction units 31-1 to 31-u to perform a simulation regarding the health of a designated employee or the like. In addition to the employee, the simulation target is not particularly limited as long as it is a target for which the past actual value (in this example, the result of the health checkup) is obtained regarding the prediction target item.

The simulation unit 32, for the specified user, if the current mode is a risk simulation mode in which the progress prediction is performed with the inquiry items (control variables) fixed, may use, as the prediction target data, a combination of explanatory variables represented by $x_{main}^{(t)}$, $\{x_{sub\_j}^{(t)}\}$, and $\{x_{ctl\_i}^{(t)}\}$, which is obtained at the current time with the current time as the first prediction reference point t in each of the prediction units 31-1 to 31-u. Further, as a result, the simulation unit 32 may obtain $x_{main}^{(t+1)}$ to $x_{main}^{(t+n)}$ corresponding to the predicted value at each prediction time point (time point t + 1 to time point t + n) included in the evaluation target period from each of the prediction units 31-1 to 31-u. At this time, the simulation unit 32 causes each of the prediction units 31 to calculate the predicted value, assuming that the value specified at each prediction time point, that is, the value set at the time point t for the value $\{x_{ctl\_i}^{(t)}\}$ of the inquiry item is maintained.

In addition, in this example, each of the prediction units 31 is configured to be able to refer to the predicted values of the other prediction units 31.

In addition, the simulation unit 32, for the specified user, if the current mode is a lifestyle habits improvement simulation mode in which the progress prediction is performed without fixing the inquiry item (control variable), may use, as the prediction target data, a combination of explanatory variables represented by $x_{main}^{(t)}$, $\{x_{sub\_j}^{(t)}\}$, and $\{\{x_{ctl\_i}^{(t)}\},...,\{x_{ctl\_i}^{(t+n-1)}\}\}$, which is obtained at the current time point with the current time point as the first prediction reference point t in each of the prediction units 31-1 to 31-u. The above description means that the value set at each prediction reference point is specified for the control variable. As a result, the simulation unit 32 may obtain the variables $x_{main}^{(t+1)}$ to $x_{main}^{(t+n)}$ from each of the prediction units 31-1 to 31-u. The value of the inquiry item may be set, for example, by accepting a user operation on the screen.

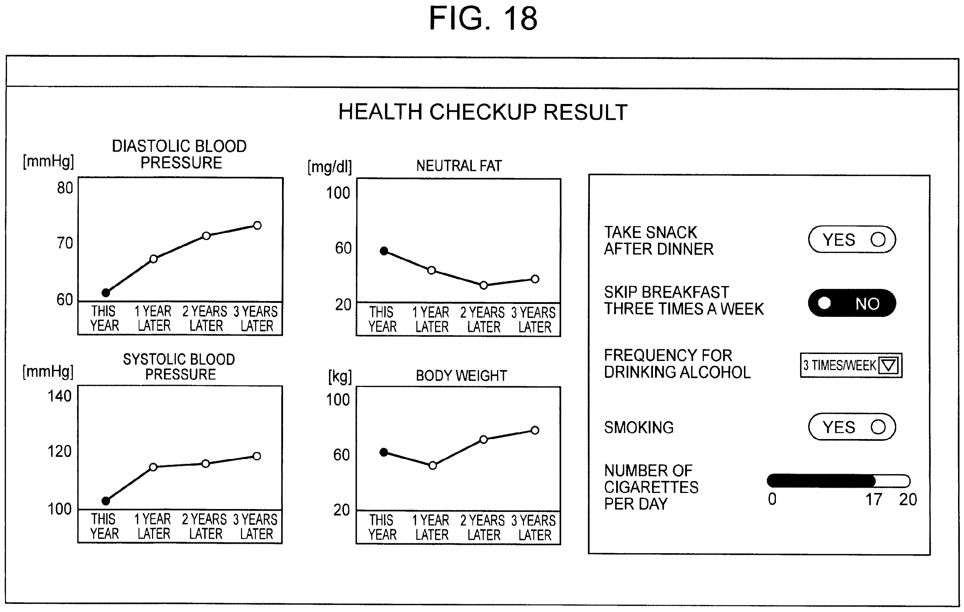

Further, the simulation unit 32 uses $x_{main}^{(t+1)}$ to $x_{main}^{(t+n)}$ obtained from each of the prediction units 31-1 to 31-u to display the predicted value at each prediction time point of each inspection item. In this example, from each of the prediction units 31-1 to 31-u, variables $x_{main}^{(t+1)}$ to $x_{main}^{(t+n)}$ corresponding to the predicted value at each prediction time point for the prediction target item corresponding to the prediction unit 31 are obtained. Fig. 18 is an explanatory diagram showing an example of a method of changing an inquiry item and a method of displaying a prediction result by the simulation unit 32. As shown in Fig. 18, the simulation unit 32 may display the transition (change over time) of the predicted value by displaying, for example, the results of the progress prediction (including at least the predicted value at each prediction time point included in the evaluation target period) in a graph for each inspection item. This allows the user to know the risk of lifestyle-related diseases when the user continues the current lifestyle habits or changes the lifestyle habits. As a result, it is possible to give advice not only to people who are currently at high risk but also to people who are expected to increase risk in the future, such as improving their lifestyle habits.

Further, the simulation unit 32 may present the transition of the predicted value obtained in the risk simulation mode and the transition of the predicted value obtained in the lifestyle habits improvement simulation mode so that they can be compared on a graph.

In addition, the simulation unit 32 can identify and present, to each of the prediction units 31 in advance, a predetermined number of high-order lifestyle habits that have a high improvement effect for each prediction target item, after comprehensively acquiring a prediction result when only one value of the inquiry item regarding lifestyle habits is changed from the value selected by the current user, while changing the inquiry item to be changed.

In each of the above exemplary embodiments, t represents a time point corresponding to the prediction unit time, but if the prediction target item changes a value by repeating some action or treatment, it is also possible that t represents the number of times the action or treatment is performed. Even in that case, in terms of expression, t may represent a time point corresponding to each time.

Next, the above-described method of calculating the invalidity score will be described in more detail. Hereinafter, the model selection unit 13 will be described as including a score calculation unit (not shown), but the model selection unit 13 can directly perform the process of the score calculating unit.

The score calculation unit inputs the series data including three or more pieces of data indicating the value of the prediction target item in association with time, at least one of these pieces of data indicating the predicted value, and calculates and outputs the invalidity score, which is an index representing the invalidity of the series data

In the following, the invalidity score is calculated as an index indicating how far the input series data is from the predetermined asymptotic model.

Here, the asymptotic model is a curve model that represents a curve having an asymptote parallel to the X-axis when time is the X-axis and the prediction item is the Y-axis, and more specifically, a curve model expressed by a function in which when $x \to \infty$, $y(x)$ converges to a certain value. Here, x represents a point (coordinate) on the time axis corresponding to each data in the series data, and $y(x)$ represents a prediction item value at the time point x. The asymptotic model may be a curve model represented by a function that satisfies at least the condition represented by the following formula (c1). Here, a is an arbitrary constant. The existing asymptote is not limited to one, and includes, for example, the one represented by a function in which two asymptotes exist such as a function called a logistic function or an arctangent function.

[Math 6]

$$\lim_{x \to \infty} y(x) = a \quad \cdot \cdot \cdot \quad (c\ 1)$$

Although the score calculation unit can also use a curve model represented by one predetermined function form as the asymptotic model, for example, the model obtained by fitting the input series data to two or more predetermined functional forms that satisfy the above conditions may be used.

The fitting may be performed, for example, by searching for a solution ($\theta$ with a hat) of a model parameter $\theta$ that minimizes a predetermined loss function as shown in the following formula (c2).

[Math 7]

$$\hat{\theta} = \text{argmin}_\theta \sum_n loss(f(x_n, \theta), y_n) \quad \cdot \cdot \cdot \quad (c\ 2)$$

$$ex)$$

$$loss(y1, y2) = (y1 - y2)^2,$$

$$f(x, \theta) = c + ba^x$$

$$\text{s.t.} \quad \theta = \{a, b, c\}, \ 0 < a < 1$$

In the formula (c2), n represents the time point of the value for which fitting is performed, loss() represents the error function, and f() represents the function form of the fitting destination. It should be noted that $f(x_n, \theta)$ represents the output when an arbitrary time point $x_n$ and a set of model parameters $\theta$ is given to the function form f(), and $f(x_n, {}^\wedge\theta)$ represents the output at an arbitrary time point $x_n$ in the asymptotic model obtained by fitting. In the example shown in the formula (c2), the square loss is used as the error function, but the error function is not limited to the square loss.

The score calculation unit may calculate, for example, an error between the asymptotic model thus obtained and the series data of the input predicted values, and output the error as an invalidity score.

The score calculation unit may output, for example, an error value (error) represented by the following formula (c3) as an invalidity score.

[Math 8]

$$error = \sum_n loss(f(x_n, \hat{\theta}), y_n) \quad \cdot \cdot \cdot \quad (c\ 3)$$

The score calculation unit may independently specify the data used for fitting and the data used for calculating the error. The score calculation unit can also accept these designations from the user. At this time, the data used for the fitting (the data belonging to the first group) and the data used for calculating the error (the data belonging to the second group) do not have to completely match.

As an example, when the series data including N piece of data is input, the fitting is performed using the first half N'(where N' < N) piece of data, and the error calculation may be performed using the value of remaining pieces of data (N-N' pieces) or all pieces of data (N pieces). In addition, as another example, it is also possible to perform fitting using data at the time points that are not continuous in the series data such as the first, third, and fifth data, and perform error calculation using all pieces of data.

For example, when series data including five pieces of data is input, the score calculation unit may perform fitting and error calculation as follows, for example.

[Example of pattern for fitting and error calculation]

- The fitting is performed with the first three pieces of data, and the error calculation is performed with the second two pieces of data.
- The fitting is performed with the first three pieces of data, and the error calculation is performed with all pieces of data.
- The fitting is performed with the first, third, and fifth data, and the error calculation is performed with all pieces of data.

Here, the number of pieces of data (the number of predicted values) Np included in the series data input to the score calculation unit is not particularly limited, but it is assumed that at least one is included. In practice, it is preferable that the series data include at least data indicating predicted values for the number of time points at which the progress prediction was performed. Note that the series data may include data indicating past actual values, and in that case, the above N represents the total number of pieces of data including data indicating past actual values. Note that N is presumed to be three or more, but from the viewpoint of fitting accuracy, for example, four or more is more preferable.

In addition, when the series data includes the data indicating the actual value, the error calculation may be performed using only the data indicating the predicted value.

In addition, the score calculation unit may rescale the x-coordinate, which is the value of the X-axis (the value representing the time corresponding to the value of each prediction item), before performing the fitting. When displaying series data as a graph, it is conceivable that the unit of a numerical value (scale width) may differ greatly between the vertical axis (Y-axis: predicted value) and the horizontal axis (X-axis: Time), such that the scale unit of the vertical axis is 50, the scale unit of the horizontal axis is 1, etc. In that case, even if the fitting is performed using the numerical values as they are, the asymptotic model expected by the viewer cannot be obtained. This is because the fitting is performed in the graph shape when the values that are greatly different in scale on the X axis and the Y axis are displayed in a graph at equal intervals, so the fitting is performed to a curve having a shape different from a curve that is a valid graph shape when actually displayed.

In order to eliminate such inconvenience, it is preferable to rescale the time value (x coordinate) associated with each data included in the series data according to the actual display, and then perform fitting.

For example, the score calculation unit, when the parameters (display parameters) related to the display such as the scale setting of the graph that displays the input series data is obtained, may convert the x coordinate so that the width of the X-axis main scale has the same unit as the width of the Y-axis main scale (50 units in the above example). In this case, whereas the unit of the width of the X axis main scale is 1, the unit of the width of the Y axis main scale is 50 (50 times), so the x coordinate is also 50 times. Hereinafter, such a scaling factor for rescaling the x axis may be referred to as a rescale parameter x_scale.

In addition, the score calculation unit can also obtain the rescale parameter x_scale as follows. The score calculation unit inputs, together with the series data, a display parameter that is a parameter when displaying the series data, and also can calculate the rescale parameter xscale, based on information obtained from the series data and the display parameter.

The following formula (c4) is an example of a formula for calculating the rescale parameter x_scale. In the formula (c4), $y_{max}$ and $y_{mim}$ represent the maximum value and the minimum value of the prediction item included in the series data, respectively. $N_d$ represents the number of pieces of data (the score of the prediction item to be displayed) included in the series data. Further, Ar represents the aspect ratio Ar (that is, the ratio of the horizontal width to the vertical width) of the display graph of the series data. In the formula (c4), 0.8 represents the display ratio in the vertical direction and 0.9 represents the display ratio in the horizontal direction, but these values are appropriately adjusted.

$$x\_scale = ((y_{max}\text{-}y_{min}) / 0.8 * Ar * 0.9) / (N_d\text{-}1)) \quad \ldots \quad (c4)$$

In the example shown in the formula (4), $y_{max}$, $y_{mim}$, and $N_d$ correspond to the information obtained from the series data, and Ar, the vertical display ratio, and the horizontal display ratio correspond to the display parameters.

For example, when the aspect ratio is 1 : 2 (Ar = 2) and five points are displayed in the frame, x_scale is calculated as follows.

$$x\_scale = ((y_{max}-y_{min}) / 0.8*2*0.9) /4)$$

In the above example, assuming that the unit of time that is associated with each data included in the series data is the prediction unit (that is, the number that increases by 1 each time the prediction time point increases by 1), x_scale is calculated as an index representing the x-direction interval of each data expressed in the unit of the y-axis. Therefore, when the unit of time in the series data is other than the prediction unit time, or when the unit of the x direction interval when displaying is other than 1, the x coordinate associated with each data may be divided by the prediction unit time or the unit of the x-direction interval to set the unit of the x axis to 1, and then multiplied by x_scale.

The score calculation unit can also accept the designation of xscale. For example, the score calculation unit may input x_scale together with the series data. Note that the score calculation unit can also calculate the x_scale by inputting the above-mentioned display parameters together with the series data.

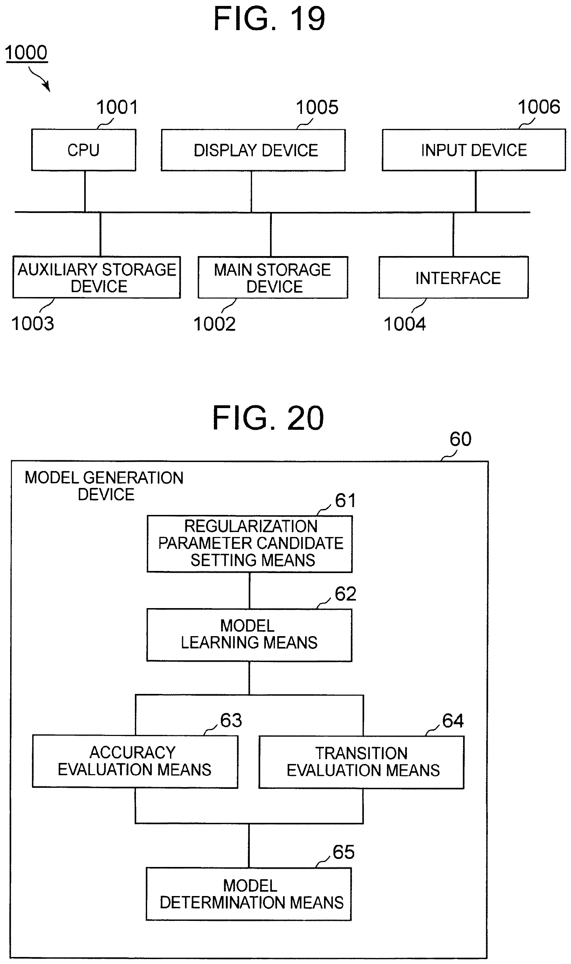

Fig. 19 is a schematic block diagram showing a configuration example of a computer according to each exemplary embodiment of the present invention. A computer 1000 includes a CPU 1001, a main storage device 1002, an auxiliary storage device 1003, an interface 1004, a display device 1005, and an input device 1006.

The server and other devices included in the prediction system and the health simulation system according to each of the above-described exemplary embodiments may be installed in the computer 1000. In that case, the operation of each device may be stored in the auxiliary storage device 1003 in the form of a program. The CPU 1001 reads the program from the auxiliary storage device 1003, expands it in the main storage device 1002, and executes the predetermined processing in each exemplary embodiment according to the program. The CPU 1001 is an example of an information processing device that operates according to a program, and may include, in addition to the CPU (Central Processing Unit), for example, MPU (Micro Processing Unit), MCU (Memory Control Unit), and GPU (Graphics Processing Unit), etc.

The auxiliary storage device 1003 is an example of a non-transitory tangible medium. Other examples of non-transitory tangible medium include a magnetic disk, a magneto-optical disk, CD-ROM, DVD-ROM, a semiconductor memory, or the like that is connected via the interface 1004. When the program is distributed to the computer 1000 through a communication line, the computer 1000 having received the distribution may expand the program into the main storage device 1002 and execute predetermined processing in each exemplary embodiment.

Further, the program may be a program for realizing a part of the predetermined processing in each exemplary embodiment. Further, the program may be a difference program that realizes predetermined processing in each exemplary embodiment in combination with another program already stored in the auxiliary storage device 1003.

The interface 1004 transmits/receives information to/from other devices. The display device 1005 also presents information to the user. Further, the input device 1006 accepts input of information from the user.

Further, depending on the processing content in the exemplary embodiment, some elements of the computer 1000 can be omitted. For example, the display device 1005 can be omitted if the computer 1000 does not present information to the user. For example, if the computer 1000 does not accept information input from the user, the input device 1006 can be omitted.

Also, some or all of the components in each of the above-described exemplary embodiments are implemented by a general-purpose or dedicated circuit (circuitry), a processor or the like, or a combination thereof. These may be configured by a single chip or may be configured by a plurality of chips connected via a bus. Some or all of the components in each of the above-described exemplary embodiments may be realized by a combination of the above-described circuitry and the like and a program.

When some or all of the components in each of the above-described exemplary embodiments are realized by a plurality of information processing devices or circuits, the plurality of information processing devices or circuits may be centrally arranged or distributed. For example, the information processing device, the circuit, and the like may be realized as a form in which a client and server system, a cloud computing system, and the like are connected to each other via a communication network.

Next, summary of the present invention will be described. Fig. 20 is a block diagram showing an outline of the model generation device of the present invention. A model generation device 60 shown in Fig. 20 includes regularization parameter candidate setting means 61, model learning means 62, accuracy evaluation means 63, transition evaluation means 64, and model determination means 65.

The regularization parameter candidate setting means 61 (for example, the regularization parameter candidate setting unit 11, the regularization parameter candidate setting unit 221) outputs a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model

The model learning means 62 (for example, the model learning unit 12, the model learning unit 222) learns a prediction model corresponding to each of a plurality of solution candidates included in the search set using the training data.

The accuracy evaluation means 63 (for example, a part of the model selection unit 13, a part of the model selection unit 223, the model evaluation unit 131), evaluates the prediction accuracy of each of the plurality of learned prediction models using the predetermined verification data.

The transition evaluation means 64 (for example, a part of the model selection unit 13, a part of the model selection unit 223, the model evaluation unit 131, the model determination unit 132) evaluates, for each of the plurality of learned prediction models, the graph shape indicated by the transition of the predicted value obtained from the prediction model or the number of defective samples, which is the number of samples for which the transition is not valid, using predetermined verification data.

The model determination means 65 (for example, a part of the model selection unit 13, a part of the model selection unit 223, the model determination unit 132) determines, based on the evaluation result regarding the prediction accuracy and the evaluation result regarding the graph shape or the number of defective samples, a single prediction model to be used for the progress prediction from among the plurality of learned prediction models.

With the above configuration, it is possible to generate a model having a good balance between the prediction accuracy and the number of defective samples. Therefore, in the progress prediction, it is possible to reduce the possibility of presenting an uninterpretable prediction result while maintaining the prediction accuracy.

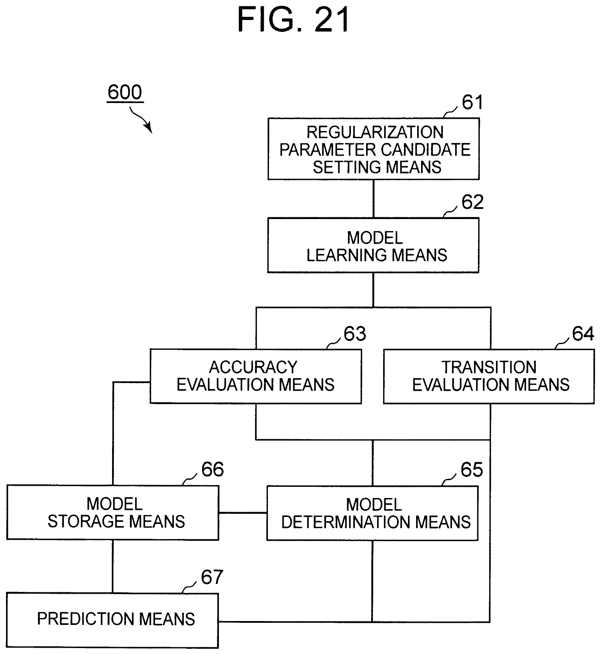

Fig. 21 is a block diagram showing the outline of the prediction system of the present invention. The prediction system shown in Fig. 21 includes model storage means 66 and prediction means 67 in addition to the components included in the model generation device 60 described above.

The model storage means 66 (for example, the model storage unit 14, the model storage unit 24) stores a prediction model used for progress prediction.

The prediction means 67 (for example, the model application unit 15, the model application unit 25), when the prediction target data is given, uses the prediction model stored in the model storage means 66 to perform progress prediction.

The above exemplary embodiment can be described as the following supplementary notes.

(Supplementary note 1) A model generation system including: regularization parameter candidate setting means that outputs a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model;

model learning means that learns, using training data, a prediction model corresponding to each of the plurality of solution candidates included in the search set; accuracy evaluation means that evaluates, using predetermined verification data, a prediction accuracy of each of a plurality of the learned prediction models; transition evaluation means that evaluates, for each of the plurality of the learned prediction models, a graph shape indicated by a transition of a predicted value obtained from the prediction model or a number of defective samples, which is a number of samples for which the transition is not valid, using predetermined verification data; and model determination means that determines a prediction model used for the progress prediction from among the plurality of the learned prediction models based on an evaluation result regarding the prediction accuracy and an evaluation result regarding the graph shape or the number of defective samples.

(Supplementary note 2) The model generation system according to supplementary note 1, in which among the explanatory variables, the strong regularization variable is a variable indicating a value of a prediction target item at an arbitrary time point before a prediction reference point or a variable indicating an arbitrary statistic calculated from the variable and another variable.

(Supplementary note 3) The model generation system according to supplementary note 1 or 2, in which the prediction model is a prediction model in which a variable indicating a value of the prediction target item at a prediction time point is a target variable, and only a main variable that is a variable indicating a value of the prediction target item at a prediction reference point, the strong regularization variable, and one or more control variables that can be controlled by a person are explanatory variables, and a value of the control variable is fixed in the progress prediction.

(Supplementary note 4) The model generation system according to any one of supplementary notes 1 to 3, in which the accuracy evaluation means obtains, for each of the plurality of the learned prediction models, an index regarding a prediction accuracy based on inspection data, the transition evaluation means obtains, for each of the plurality of the learned prediction models, an index regarding the graph shape or the number of defective samples based on the inspection data, and the model determination means determines a prediction model used for the progress prediction based on the index regarding the prediction accuracy and the index regarding the graph shape or the number of defective samples.

(Supplementary note 5) A model generation system including: constrained model evaluation means that evaluates, using predetermined verification data, a prediction accuracy of a constrained model, which is one of prediction models used for progress prediction performed by fixing some values of a plurality of explanatory variables, which is a prediction model that predicts a value of a prediction target item at a prediction time point when a predicted value is obtained, and which is a prediction model in which at least a constraint that a variable other than a main variable indicating a value of the prediction target item at a prediction reference point is not used as a non-control variable that is an explanatory variable whose value changes in the progress prediction is imposed to the explanatory variable; regularization parameter candidate setting means that outputs a search set which is a search set of regularization parameters used for regularization of a calibration model, and in which a plurality of solution candidates are set, the solution candidates having at least mutually different values of a regularization parameter that affects a term of a strong regularization variable that is one or more variables specifically defined among the explanatory variables used for a model formula of the calibration model, the calibration model being one of the prediction models used for the progress prediction, the calibration model being a prediction model for predicting a calibration value for calibrating the predicted value obtained in the constrained model for arbitrary prediction target data, and the calibration model being a prediction model including two or more non-control variables and one or more control variables that can be controlled by a person in the explanatory variables; model learning means that learns, using training data, a calibration model corresponding to each of the plurality of solution candidates included in the search set; accuracy evaluation means that evaluates, using predetermined verification data, a prediction accuracy of each of the plurality of learned calibration models; transition evaluation means that evaluates, for each of the plurality of learned calibration models, a graph shape indicated by a transition of the predicted value after calibration obtained as a result of calibrating the predicted value obtained from the constrained model with the calibration value obtained from the calibration model or the number of defective samples, which is the number of samples for which the transition is not valid, using predetermined verification data; and model determination means that determines a calibration model used for the progress prediction from among the plurality of learned calibration models based on an index regarding the prediction accuracy and an index regarding the graph shape or the number of defective samples.

(Supplementary note 6) The model generation system according to any one of supplementary notes 1 to 5, in which the index regarding the graph shape is an invalidity score calculated based on an error between a curve model obtained by fitting series data into a predetermined function form and the series data, the series data including data indicating a predicted value at each prediction time point obtained by the previous progress prediction, and the series data including three or more pieces of data indicating a value of the prediction target item in association with time.

(Supplementary note 7) A prediction system including: regularization parameter candidate setting means that outputs a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model; model learning means that learns, using training data, a prediction model corresponding to each of the plurality of solution candidates included in the search set; accuracy evaluation means that evaluates, using predetermined verification data, a prediction accuracy of each of a plurality of the learned prediction models; transition evaluation means that evaluates, for each of the plurality of the learned prediction models, a graph shape indicated by a transition of a predicted value obtained from the prediction model or a number of defective samples, which is a number of samples for which the transition is not valid, using predetermined verification data; model determination means that determines a prediction model used for the progress prediction from among the plurality of the learned prediction models based on an evaluation result regarding the prediction accuracy and an evaluation result regarding the graph shape or the number of defective samples; model storage means that stores a prediction model used for the progress prediction; and prediction means that when prediction target data is given, performs the progress prediction using the prediction model stored in the model storage means.

(Supplementary note 8) The prediction system according to supplementary note 7, in which the model determination means determines, before the progress prediction is performed, a prediction model used for the progress prediction, and the model storage means stores the prediction model determined by the model determination means.

(Supplementary note 9) The prediction system according to supplementary note 7, in which the model storage means stores a plurality of the prediction models learned by the model learning means, the prediction means performs the progress prediction using each of the plurality of prediction models stored in the model storage means to acquire a predicted value at each prediction time point in a period targeted for the current progress prediction from each of the prediction models, the transition evaluation means, based on verification data including prediction target data in the current progress prediction, evaluates a graph shape at the time of the past progress prediction including the time of the current progress prediction, or the number of defective samples at the time of the past progress prediction including the time of the current progress prediction, and the model determination means, based on the index regarding the prediction accuracy and the index regarding the graph shape or the number of defective samples, determines a prediction model to use for the predicted value at each prediction time point in the current progress prediction from among the plurality of prediction models stored in the model storage means.

(Supplementary note 10) The prediction system according to any one of supplementary notes 7 to 9, further including shipping determination means that performs, when series data is input, evaluation regarding at least the graph shape or the number of defective samples on the series data, a predicted value included in the series data, or a prediction model that has obtained the predicted value, and performs shipping determination of the predicted value based on the evaluation result, the series data being the index regarding the graph shape that includes data indicating a predicted value at each prediction time point obtained by the previous progress prediction, and the series data including two or more pieces of data indicating a value of the prediction target item in association with time.

(Supplementary note 11) A model generation method including: outputting a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model; learning, using training data, a prediction model corresponding to each of the plurality of solution candidates included in the search set; evaluating, for each of the plurality of the learned prediction models, each of a prediction accuracy and a graph shape indicated by a transition of a predicted value obtained from the prediction model or a number of defective samples, which is a number of samples for which the transition is not valid, using predetermined verification data; and determining a prediction model used for the progress prediction from among the plurality of the learned prediction models based on an evaluation result regarding the prediction accuracy and an evaluation result regarding the graph shape or the number of defective samples.

(Supplementary note 12) A model generation program for causing a computer to execute the processes of: outputting a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model; learning, using training data, a prediction model corresponding to each of the plurality of solution candidates included in the search set; evaluating, for each of the plurality of the learned prediction models, each of a prediction accuracy and a graph shape indicated by a transition of a predicted value obtained from the prediction model or a number of defective samples, which is a number of samples for which the transition is not valid, using predetermined verification data; and determining a prediction model used for the progress prediction from among the plurality of the learned prediction models based on an evaluation result regarding the prediction accuracy and an evaluation result regarding the graph shape or the number of defective samples.

Although the present invention has been described above with reference to the exemplary embodiments and examples, the present invention is not limited to the above-described exemplary embodiments and examples. Various modifications that can be understood by those skilled in the art can be made to the configuration and details of the present invention within the scope of the present invention.

This application claims the priority on the basis of Japanese patent application 2018-068278 for which it applied on March 30, 2018, and takes in its entirety of the disclosure herein.

Industrial Applicability

The present invention can be suitably applied to a thing that obtains a predicted value at each prediction time point included in a predetermined evaluation target period specified as a progress prediction target and including one or more prediction time points by fixing some values of a plurality of explanatory variables.

Reference Signs List

| | |
|---|---|
| 100, 200 | Prediction system |
| 10, 10A | Model generation unit |
| 11 | Regularization parameter candidate setting unit |
| 12 | Model learning unit |
| 13 | Model selection unit |
| 131 | Model evaluation unit |
| 132 | Model determination unit |
| 14, 24 | Model storage unit |
| 15, 25 | Model application unit |
| 21 | Constrained model generation unit |
| 211 | Constraint imparting unit |
| 212 | Model learning unit |
| 213 | Model evaluation unit |
| 22 | Calibration model generation unit |
| 221 | Regularization parameter candidate setting unit |
| 222 | Model learning unit |
| 223 | Model selection unit |
| 251 | Constrained model application unit |
| 252 | Calibration model application unit |
| 253 | Calibration unit |
| 23 | Data processing unit |
| 30 | Health simulation system |
| 31 | Prediction unit |
| 32 | Simulation unit |
| 1000 | Computer |
| 1001 | CPU |
| 1002 | Main storage device |
| 1003 | Auxiliary storage device |
| 1004 | Interface |
| 1005 | Display device |
| 1006 | Input device |
| 60 | Model generation device |
| 600 | Prediction system |
| 61 | Regularization parameter candidate setting means |
| 62 | Model learning means |
| 63 | Accuracy evaluation means |
| 64 | Transition evaluation means |
| 65 | Model determination means |
| 66 | Model storage means |
| 67 | Prediction means |

## Claims

1. A model generation system comprising:

regularization parameter candidate setting means that outputs a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model;
model learning means that learns, using training data, a prediction model corresponding to each of the plurality of solution candidates included in the search set;

accuracy evaluation means that evaluates, using predetermined verification data, a prediction accuracy of each of a plurality of the learned prediction models;

transition evaluation means that evaluates, for each of the plurality of the learned prediction models, a graph shape indicated by a transition of a predicted value obtained from the prediction model or a number of defective samples, which is a number of samples for which the transition is not valid, using predetermined verification data; and

model determination means that determines a prediction model used for the progress prediction from among the plurality of the learned prediction models based on an evaluation result regarding the prediction accuracy and an evaluation result regarding the graph shape or the number of defective samples.

2. The model generation system according to claim 1, wherein
among the explanatory variables, the strong regularization variable is a variable indicating a value of a prediction target item at an arbitrary time point before a prediction reference point or a variable indicating an arbitrary statistic calculated from the variable and another variable.

3. The model generation system according to claim 1 or 2, wherein
the prediction model is a prediction model in which a variable indicating a value of the prediction target item at a prediction time point is a target variable, and only a main variable that is a variable indicating a value of the prediction target item at a prediction reference point, the strong regularization variable, and one or more control variables that can be controlled by a person are explanatory variables, and
a value of the control variable is fixed in the progress prediction.

4. The model generation system according to any one of claims 1 to 3, wherein
the accuracy evaluation means obtains, for each of the plurality of the learned prediction models, an index regarding a prediction accuracy based on inspection data,
the transition evaluation means obtains, for each of the plurality of the learned prediction models, an index regarding the graph shape or the number of defective samples based on the inspection data, and
the model determination means determines a prediction model used for the progress prediction based on the index regarding the prediction accuracy and the index regarding the graph shape or the number of defective samples.

5. A model generation system comprising:

constrained model evaluation means that evaluates, using predetermined verification data, a prediction accuracy of a constrained model, which is one of prediction models used for progress prediction performed by fixing some values of a plurality of explanatory variables, which is a prediction model that predicts a value of a prediction target item at a prediction time point when a predicted value is obtained, and which is a prediction model in which at least a constraint that a variable other than a main variable indicating a value of the prediction target item at a prediction reference point is not used as a non-control variable that is an explanatory variable whose value changes in the progress prediction is imposed to the explanatory variable;

regularization parameter candidate setting means that outputs a search set which is a search set of regularization parameters used for regularization of a calibration model, and in which a plurality of solution candidates are set, the solution candidates having at least mutually different values of a regularization parameter that affects a term of a strong regularization variable that is one or more variables specifically defined among the explanatory variables used for a model formula of the calibration model, the calibration model being one of the prediction models used for the progress prediction, the calibration model being a prediction model for predicting a calibration value for calibrating the predicted value obtained in the constrained model for arbitrary prediction target data, and the calibration model being a prediction model including two or more non-control variables and one or more control variables that can be controlled by a person in the explanatory variables;

model learning means that learns, using training data, a calibration model corresponding to each of the plurality of solution candidates included in the search set;

accuracy evaluation means that evaluates, using predetermined verification data, a prediction accuracy of each of the plurality of learned calibration models;

transition evaluation means that evaluates, for each of the plurality of learned calibration models, a graph shape indicated by a transition of the predicted value after calibration obtained as a result of calibrating the predicted value obtained from the constrained model with the calibration value obtained from the calibration model or the number of defective samples, which is the number of samples for which the transition is not valid, using predetermined verification data; and

model determination means that determines a calibration model used for the progress prediction from among the plurality of learned calibration models based on an index regarding the prediction accuracy and an index regarding the graph shape or the number of defective samples.

6. The model generation system according to any one of claims 1 to 5, wherein

the index regarding the graph shape is an invalidity score calculated based on an error between a curve model obtained by fitting series data into a predetermined function form and the series data, the series data including data indicating a predicted value at each prediction time point obtained by the previous progress prediction, and the series data including three or more pieces of data indicating a value of the prediction target item in association with time.

7. A prediction system comprising:

regularization parameter candidate setting means that outputs a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model;

model learning means that learns, using training data, a prediction model corresponding to each of the plurality of solution candidates included in the search set;

accuracy evaluation means that evaluates, using predetermined verification data, a prediction accuracy of each of a plurality of the learned prediction models;

transition evaluation means that evaluates, for each of the plurality of the learned prediction models, a graph shape indicated by a transition of a predicted value obtained from the prediction model or a number of defective samples, which is a number of samples for which the transition is not valid, using predetermined verification data;

model determination means that determines a prediction model used for the progress prediction from among the plurality of the learned prediction models based on an evaluation result regarding the prediction accuracy and an evaluation result regarding the graph shape or the number of defective samples;

model storage means that stores a prediction model used for the progress prediction; and

prediction means that when prediction target data is given, performs the progress prediction using the prediction model stored in the model storage means.

8. The prediction system according to claim 7, wherein

the model determination means determines, before the progress prediction is performed, a prediction model used for the progress prediction, and

the model storage means stores the prediction model determined by the model determination means.

9. The prediction system according to claim 7, wherein

the model storage means stores a plurality of the prediction models learned by the model learning means,

the prediction means performs the progress prediction using each of the plurality of prediction models stored in the model storage means to acquire a predicted value at each prediction time point in a period targeted for the current progress prediction from each of the prediction models,

the transition evaluation means, based on verification data including prediction target data in the current progress prediction, evaluates a graph shape at the time of the past progress prediction including the time of the current progress prediction, or the number of defective samples at the time of the past progress prediction including the time of the current progress prediction, and

the model determination means, based on the index regarding the prediction accuracy and the index regarding the graph shape or the number of defective samples, determines a prediction model to use for the predicted value at each prediction time point in the current progress prediction from among the plurality of prediction models stored in the model storage means.

10. The prediction system according to any one of claims 7 to 9, further comprising

shipping determination means that performs, when series data is input, evaluation regarding at least the graph shape or the number of defective samples on the series data, a predicted value included in the series data, or a prediction model that has obtained the predicted value, and performs shipping determination of the predicted value based on the evaluation result, the series data being the index regarding the graph shape that includes data indicating a predicted value at each prediction time point obtained by the previous progress prediction, and the series data including two or more pieces of data indicating a value of the prediction target item in association with time.

**11.** A model generation method comprising:

outputting a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model;

learning, using training data, a prediction model corresponding to each of the plurality of solution candidates included in the search set;

evaluating, for each of the plurality of the learned prediction models, each of a prediction accuracy and a graph shape indicated by a transition of a predicted value obtained from the prediction model or a number of defective samples, which is a number of samples for which the transition is not valid, using predetermined verification data; and

determining a prediction model used for the progress prediction from among the plurality of the learned prediction models based on an evaluation result regarding the prediction accuracy and an evaluation result regarding the graph shape or the number of defective samples.

**12.** A model generation program for causing a computer to execute the processes of:

outputting a search set which is a search set of regularization parameters used for regularization of a prediction model used for progress prediction performed by fixing some values of a plurality of explanatory variables, and in which a plurality of solution candidates are set, the solution candidates having at least mutually different values of a regularization parameter that affects a term of a strong regularization variable that is one or more variables specifically defined among the explanatory variables used for a prediction formula of the prediction model;

learning, using training data, a prediction model corresponding to each of the plurality of solution candidates included in the search set;

evaluating, for each of the plurality of the learned prediction models, each of a prediction accuracy and a graph shape indicated by a transition of a predicted value obtained from the prediction model or a number of defective samples, which is a number of samples for which the transition is not valid, using predetermined verification data; and

determining a prediction model used for the progress prediction from among the plurality of the learned prediction models based on an evaluation result regarding the prediction accuracy and an evaluation result regarding the graph shape or the number of defective samples.

# FIG. 1A

EVALUATION TARGET PERIOD
(WHEN N = 3, n = 3)

PREDICTION
TIME UNIT

ACTUAL VALUE EXISTS

TIME

| TIME POINT IMMEDIATELY BEFORE (t = -1) | REFERENCE POINT (t = 0) | FIRST TIME POINT (t = 1) | SECOND TIME POINT (t = 2) | THIRD TIME POINT (t = 3) |

FIRST PREDICTION REFERENCE POINT / FIRST PREDICTION TIME POINT / SECOND PREDICTION TIME POINT / THIRD PREDICTION TIME POINT

# FIG. 1B

EVALUATION TARGET PERIOD
(WHEN N = 3, n = 2)

PREDICTION
TIME UNIT

ACTUAL VALUE EXISTS

TIME

| TIME POINT IMMEDIATELY BEFORE (t = -1) | REFERENCE POINT (t = 0) | FIRST TIME POINT (t = 1) | SECOND TIME POINT (t = 2) | THIRD TIME POINT (t = 3) |

FIRST PREDICTION REFERENCE POINT / FIRST PREDICTION TIME POINT / SECOND PREDICTION TIME POINT

# FIG. 2A

$x^{(t)}$ → PREDICTION MODEL → $y^{(t+1)}$

# FIG. 2B

$x_1^{(2)} \leftarrow y^{(2)}$

$x_1^{(1)} \leftarrow y^{(1)}$

$x^{(0)}$
$x^{(1)}$   →   PREDICTION MODEL   →   $y^{(1)}$
$x^{(2)}$                                        $y^{(2)}$
                                                 $y^{(3)}$

# FIG. 3

PREDICTION SYSTEM

MODEL GENERATION UNIT

TRAINING DATA → REGULARIZATION PARAMETER CANDIDATE SETTING UNIT — 11

MODEL LEARNING UNIT — 12

MODEL SELECTION UNIT — 13

— 10

MODEL STORAGE UNIT — 14

PREDICTION TARGET DATA → MODEL APPLICATION UNIT — 15 → PREDICTED VALUE AND ITS TRANSITION

100

# FIG. 4

MODEL LEARNING PHASE

START

INPUT TRAINING DATA — S101

DETERMINE SEARCH SET (PLURALITY OF SOLUTION CANDIDATES) FOR REGULARIZATION PARAMETER — S102

LEARN MODEL FOR EACH SOLUTION CANDIDATE — S103

EVALUATE LEARNED MODELS AND SELECT AND SAVE ONE MODEL — S104

END

# FIG. 5

PREDICTION PHASE

( START )

↓

INPUT PREDICTION TARGET DATA ⟵ S201

↓

READ STORED MODEL ⟵ S202

↓

APPLY PREDICTION TARGET DATA
TO MODEL TO OBTAIN PREDICTED ⟵ S203
VALUE AND ITS TRANSITION

↓

( END )

# FIG. 6A

CONFIRMATION PERIOD
(N = 3, n = 3)

INSPECTION VALUE

PREDICTION TIME POINT 1
PREDICTION TIME POINT 2
PREDICTION TIME POINT 3

APPROXIMATE CURVE

0    1    2    3    TIME POINT t

# FIG. 6B

CONFIRMATION PERIOD
(N = 3, n = 3)

INSPECTION VALUE

PREDICTION TIME POINT 1
PREDICTION TIME POINT 2
PREDICTION TIME POINT 3

APPROXIMATE CURVE

0    1    2    3    TIME POINT t

# FIG. 7

# FIG. 8A

# FIG. 8B

# FIG. 9

PREDICTION MODEL (FULL EXPRESSION)

$$y^{(t+1)} = w_0 + w_m x_{main}^{(t)} + \underbrace{w_{r\_1} x_{reg\_1}^{(t)} + \cdots + w_{r\_p} x_{reg\_p}^{(t)}}_{\varphi^T x_{reg}^{(t)}} + \underbrace{w_{c\_1} x_{clt\_1}^{(t)} + \cdots + w_{c\_q} x_{ctl\_q}^{(t)}}_{\theta^T x_{ctl}^{(t)}}$$

PREDICTION MODEL (SIMPLIFIED EXPRESSION)

$$y^{(t+1)} = w_m x_{main}^{(t)} + \varphi^T x_{reg}^{(t)} + \theta^T x_{ctl}^{(t)} + w_0$$

EP 3 780 003 A1

# FIG. 10

PREDICTION SYSTEM

MODEL GENERATION UNIT

TRAINING DATA — REGULARIZATION PARAMETER CANDIDATE SETTING UNIT ~11 — MODEL LEARNING UNIT ~12

MODEL EVALUATION UNIT ~131

10A

MODEL STORAGE UNIT ~14

PREDICTION TARGET DATA — MODEL APPLICATION UNIT ~15

MODEL DETERMINATION UNIT ~132 — PREDICTED VALUE AND ITS TRANSITION

100

# FIG. 11

PREDICTION SYSTEM

CONSTRAINED MODEL GENERATION UNIT

TRAINING DATA 1 → CONSTRAINT IMPARTING UNIT — MODEL LEARNING UNIT ~212

211

MODEL EVALUATION UNIT ~213  ~21

CALIBRATION MODEL GENERATION UNIT

TRAINING DATA 2 → REGULARIZATION PARAMETER CANDIDATE SETTING UNIT — MODEL LEARNING UNIT ~222  ~22

221

MODEL SELECTION UNIT ~223

MODEL STORAGE UNIT

24

PREDICTION TARGET DATA → MODEL APPLICATION UNIT → PREDICTED VALUE AND ITS TRANSITION

25

200

EP 3 780 003 A1

# FIG. 12A

$0 < a < 1$

y

n

# FIG. 12B

$1 < a$

y

n

# FIG. 12C

$-1 < a < 0$

y

n

# FIG. 12D

$a < -1$

y

n

# FIG. 13

PREDICTION
TARGET
DATA

MODEL
APPLICATION UNIT

CONSTRAINED
MODEL
APPLICATION UNIT
251

CALIBRATION
MODEL
APPLICATION UNIT
252

CALIBRATION
UNIT
253

PREDICTED
VALUE
AND ITS
TRANSITION

25

# FIG. 14

MODEL LEARNING PHASE

START

INPUT TRAINING DATA 1 ～S301

GENERATE CONSTRAINED MODEL ～S302

SAVE CONSTRAINED MODEL ～S303

EVALUATION OF CONSTRAINED MODEL
(GENERATION, INPUT OF
TRAINING DATA 2) ～S304

DETERMINE SEARCH SET
(PLURALITY OF SOLUTION CANDIDATES)
OF REGULARIZATION PARAMETER OF
CALIBRATION MODEL ～S305

LEARN CALIBRATION MODEL FOR
EACH SOLUTION CANDIDATE ～S306

SELECT AND SAVE ONE FROM AMONG
LEARNED CALIBRATION MODELS ～S307

END

# FIG. 15

PREDICTION PHASE

START

INPUT PREDICTION TARGET DATA — S401

S411
READ STORED
CONSTRAINED MODEL

S421
READ STORED
CALIBRATION MODEL

S412
APPLY PREDICTION TARGET DATA TO
CONSTRAINED MODEL TO OBTAIN
PREDICTED VALUE AT EACH
PREDICTION TIME POINT

S422
APPLY PREDICTION TARGET DATA TO
CALIBRATION MODEL TO OBTAIN
CALIBRATION VALUE AT EACH
PREDICTION TIME POINT

S423
CALIBRATE EACH PREDICTED VALUE
WITH CALIBRATION VALUE TO OBTAIN
FINAL PREDICTED VALUE

END

# FIG. 16

PREDICTION SYSTEM

CONSTRAINED MODEL GENERATION UNIT — 21

TRAINING DATA

CONSTRAINT IMPARTING UNIT — 211

MODEL LEARNING UNIT — 212

MODEL EVALUATION UNIT — 213

DATA PROCESSING UNIT — 23

CALIBRATION MODEL GENERATION UNIT

REGULARIZATION PARAMETER CANDIDATE SETTING UNIT — 221

MODEL LEARNING UNIT — 222

MODEL SELECTION UNIT — 223

MODEL STORAGE UNIT — 24

— 22

PREDICTION TARGET DATA

MODEL APPLICATION UNIT — 25

PREDICTED VALUE AND ITS TRANSITION

200

EP 3 780 003 A1

# FIG. 17

HEALTH SIMULATION SYSTEM

PREDICTION UNIT
31-1

PREDICTION UNIT
31-u

SIMULATION UNIT
32

30

# FIG. 18

## HEALTH CHECKUP RESULT

### DIASTOLIC BLOOD PRESSURE

[mmHg]

80, 70, 60 — THIS YEAR, 1 YEAR LATER, 2 YEARS LATER, 3 YEARS LATER

### NEUTRAL FAT

[mg/dl]

100, 60, 20 — THIS YEAR, 1 YEAR LATER, 2 YEARS LATER, 3 YEARS LATER

### SYSTOLIC BLOOD PRESSURE

[mmHg]

140, 120, 100 — THIS YEAR, 1 YEAR LATER, 2 YEARS LATER, 3 YEARS LATER

### BODY WEIGHT

[kg]

100, 60, 20 — THIS YEAR, 1 YEAR LATER, 2 YEARS LATER, 3 YEARS LATER

TAKE SNACK AFTER DINNER — YES ○

SKIP BREAKFAST THREE TIMES A WEEK — ● NO

FREQUENCY FOR DRINKING ALCOHOL — 3 TIMES/WEEK ▽

SMOKING — YES ○

NUMBER OF CIGARETTES PER DAY — 0 ▬▬▬ 17 20

EP 3 780 003 A1

# FIG. 19

1000

| 1001 | 1005 | 1006 |
|------|------|------|
| CPU | DISPLAY DEVICE | INPUT DEVICE |

| AUXILIARY STORAGE DEVICE | MAIN STORAGE DEVICE | INTERFACE |
|------|------|------|
| 1003 | 1002 | 1004 |

# FIG. 20

60

MODEL GENERATION DEVICE

61
REGULARIZATION PARAMETER CANDIDATE SETTING MEANS

62
MODEL LEARNING MEANS

63
ACCURACY EVALUATION MEANS

64
TRANSITION EVALUATION MEANS

65
MODEL DETERMINATION MEANS

# FIG. 21

_600_

REGULARIZATION
PARAMETER CANDIDATE
SETTING MEANS — 61

MODEL
LEARNING MEANS — 62

ACCURACY
EVALUATION MEANS — 63

TRANSITION
EVALUATION MEANS — 64

MODEL
STORAGE MEANS — 66

MODEL
DETERMINATION MEANS — 65

PREDICTION MEANS — 67

# FIG. 22A

# FIG. 22B

# FIG. 23A

INSPECTION VALUE

1 YEAR LATER

THIS YEAR (ACTUAL VALUE)

2 YEARS LATER

LAST YEAR (ACTUAL VALUE)

TIME

# FIG. 23B

INSPECTION VALUE

LAST YEAR (ACTUAL VALUE)

2 YEARS LATER

THIS YEAR (ACTUAL VALUE)

1 YEAR LATER

TIME

# FIG. 23C

INSPECTION VALUE

LAST YEAR (ACTUAL VALUE)

1 YEAR LATER

THIS YEAR (ACTUAL VALUE)

2 YEARS LATER

TIME

## INTERNATIONAL SEARCH REPORT

| | International application No. |
|---|---|
| | PCT/JP2018/045610 |

| A. CLASSIFICATION OF SUBJECT MATTER |
|---|
| Int. Cl. G16H50/50(2018.01)i |

According to International Patent Classification (IPC) or to both national classification and IPC

| B. FIELDS SEARCHED |
|---|
| Minimum documentation searched (classification system followed by classification symbols) |
| Int. Cl. G16H50/50 |

Documentation searched other than minimum documentation to the extent that such documents are included in the fields searched

```
Published examined utility model applications of Japan      1922-1996
Published unexamined utility model applications of Japan    1971-2019
Registered utility model specifications of Japan            1996-2019
Published registered utility model applications of Japan    1994-2019
```

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| A | JP 2018-010475 A (FUJITSU LTD.) 18 January 2018, paragraphs [0002]-[0081], fig. 1 & US 2018/018586 A1, paragraphs [0003]-[0087], fig. 1 | 1-12 |
| A | JP 2016-146046 A (JSOL CORP.) 12 August 2016, paragraphs [0060]-[0089], fig. 1 (Family: none) | 1-12 |
| A | WO 2017/098631 A1 (MITSUBISHI ELECTRIC CORP.) 15 June 2017, paragraphs [0028]-[0078], fig. 3A & US 2018/262003 A1, paragraphs [0036]-[0086], fig. 3A & EP 3389162 A1 & CN 108292860 A | 1-12 |

☐ Further documents are listed in the continuation of Box C.  ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 08.03.2019 | 19.03.2019 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012064087 A
- JP 2014225175 A
- JP 2018068278 A